# EUROPEAN PATENT APPLICATION

(11) **EP 4 846 919 A1**
(43) Date of publication of application: **02.12.2026**
(21) Application number: 25745059.3
(22) Date of filing: 21.01.2025
(51) Int. Cl.: A61B 1/008, A61B 1/00

(54) **INSERTION APPARATUS**

(30) Priority: 23.01.2024 US 202463623861 P
(71) Applicant: Olympus Medical Systems Corp., Tokyo 192-8507 (JP)
(72) Inventor: KANETA, Giichi, Hachioji-shi, Tokyo 192-8507 (JP); SUZUKI, Hisanobu, Hachioji-shi, Tokyo 192-8507 (JP); UEHARA, Shohei, Hachioji-shi, Tokyo 192-8507 (JP)
(74) Representative: Schicker, Silvia
(86) International application number: PCT/JP2025/001727
(87) International publication number: WO 2025/159075

(57) **Abstract**

An endoscope includes a pair of vertical bending operation wires for transmitting a pulling force to a bending portion of an insertion portion, a vertical bending pulley including a pair of pulley grooves on an outer circumference thereof, and wire guides that are provided in a partial region in a circumference direction of each of the pulley grooves and guide the vertical bending operation wires to be wound in the pulley grooves respectively. An angle within which the vertical bending pulley can rotate is 180 degrees or less, and a bottom portion of each of the pulley grooves has a width that allows each of the vertical bending operation wires to be wound for at most two rounds. The width of the bottom portion of the pulley groove is narrowed down with each of the wire guides so that each of the vertical bending operation wires can be wound only one round in a rotation axis direction of each of the vertical bending pulleys, and each of the wire guides has a tapered shape to guide each of the vertical bending operation wires to be wound in the partial region to the bottom portion of each of the pulley grooves.

## Description

### Technical Field

The present invention relates to an insertion device that causes a bending portion to bend via a bending operation wire that is wound on a pulley.

### Background Art

Conventionally, endoscopes, which are one form of insertion devices, have been widely used in the medical field. The endoscope includes an elongated insertion portion. By inserting the insertion portion into a lumen, which is a subject, it is possible to observe an inside of the lumen using the endoscope.

In an endoscope including a bending portion in an insertion portion, a bending operation to the bending portion is performed by using a bending operation mechanism provided in an operation portion. The bending operation mechanism includes a pulley rotatably provided inside the operation portion, and a bending operation wire that connects the pulley to the bending portion. Such a bending operation mechanism changes a winding amount of the bending operation wire in a groove of the pulley according to a rotating state of the pulley. With the change in the winding amount, the bending operation mechanism can bend the bending portion (see Japanese Patent Application Laid-Open Publication No. 2010-119556, for example).

### Citation List

### Patent Document

Patent Document 1: Japanese Patent Application Laid-Open Publication No. 2010-119556

### Summary of the Invention

### Problem to be Solved by the Invention

However, a bending operation wire used in an insertion device such as an endoscope has predetermined elasticity. Accordingly, when a pulley is rotated in a direction in which the bending operation wire is relaxed, the bending operation wire displaces in a direction away from a groove of the pulley, due to an elastic restoring force of the bending operation wire. If the pulley is rotated in a direction in which the bending operation wire is pulled after such displacement of the bending operation wire occurs, there is a possibility that it is difficult to wind the bending operation wire in the groove of the pulley in an appropriate state. For example, in a bending operation mechanism in which the bending operation wire can be wound in the groove of the pulley one or more rounds, there is a possibility that the bending operation wire is wound in the groove in a state where the bending operation wire overlaps in a radial direction of the pulley.

An objective of the present invention is to provide an insertion device capable of winding a bending operation wire in a groove of a pulley in an appropriate state.

### Means for Solving the Problem

An insertion device according to an aspect of the present invention includes: a wire configured to transmit a pulling force to a bending portion of an insertion portion configured to be inserted into a subject; a pulley including a groove for winding the wire on an outer circumference portion of the pulley; and a wire guide that is provided in a partial region in a circumference direction of the groove, and is configured to guide the wire to be wound in the groove to a predetermined winding position. An angle range within which the pulley can rotate is from -180 degrees to +180 degrees with respect to an initial position of the pulley. A width of a bottom portion of the groove in the partial region is narrowed down with the wire guide so that the wire can be wound only one round in the direction of the rotation axis of the pulley, and the wire guide has a tapered shape to guide the wire to be wound in the partial region to the bottom portion of the groove.

An insertion device according to another aspect of the present invention includes: a first wire, a second wire, a third wire, and a fourth wire each configured to transmit a pulling force to a bending portion of an insertion portion configured to be inserted into a subject; a first pulley and a second pulley, the first pulley including a first groove and a second groove for winding the first wire and the second wire respectively, on an outer circumference portion of the first pulley, the second pulley including a third groove and a fourth groove for winding the third wire and the fourth wire respectively, on an outer circumference portion of the second pulley; and a first wire guide, a second wire guide, a third wire guide, and a fourth wire guide that are provided in a partial region in a circumference direction of each of the first groove, the second groove, the third groove, and the fourth groove, and are configured to respectively guide the first wire, the second wire, the third wire, and the fourth wire to be wound in the first groove, the second groove, the third groove, and the fourth groove, respectively, to predetermined winding positions. Angle ranges within which the first pulley and the second pulley can rotate are from -180 degrees to +180 degrees with respect to respective initial positions of the first pulley and the second pulley. Widths of bottom portions of the first groove, the second groove, the third groove, and the fourth groove in the partial region are narrowed down with the first wire guide, the second wire guide, the third wire guide, and the fourth wire guide, respectively, so that the first wire, the second wire, the third wire, and the fourth wire can be wound only one round in the direction of the rotation axis of the first pulley or the second pulley, and the first wire guide, the second wire guide, the third wire guide, and the fourth wire guide have a tapered shape to guide the first wire, the second wire, the third wire, and the fourth wire to be wound in the partial region to the bottom portions of the first groove, the second groove, the third groove, and the fourth groove respectively.

### Effects of the Invention

According to the insertion device of the present invention, it is possible to wind a bending operation wire in a groove of a pulley in an appropriate state.

### Brief Description of the Drawings

Fig. 1 is a perspective view showing an endoscope.
Fig. 2 is a perspective view showing a distal end portion.
Fig. 3 is an exploded perspective view of an operation portion.
Fig. 4 is a sectional view of a main part of a bending portion.
Fig. 5 is an end view of the bending portion when seen from a distal end side.
Fig. 6 is a perspective view showing a distal end side of an outer sheath.
Fig. 7 is a sectional view of the main part of the bending portion when bent.
Fig. 8 is a sectional view of a main part of a bending operation mechanism.
Fig. 9 is an exploded perspective view showing a bending operation mechanism main body from one end side.
Fig. 10 is an exploded perspective view showing the bending operation mechanism main body from another end side.
Fig. 11 is a sectional view showing a vertical bending pulley and a protective member taken along the line XI-XI in Fig. 8.
Fig. 12 is an exploded sectional view showing the vertical bending pulley and the protective member in Fig. 11.
Fig. 13 is a sectional view showing a lateral bending pulley and a protective member taken along the line XIII-XIII in Fig. 8.
Fig. 14 is an exploded sectional view showing the lateral bending pulley and the protective member in Fig. 13.

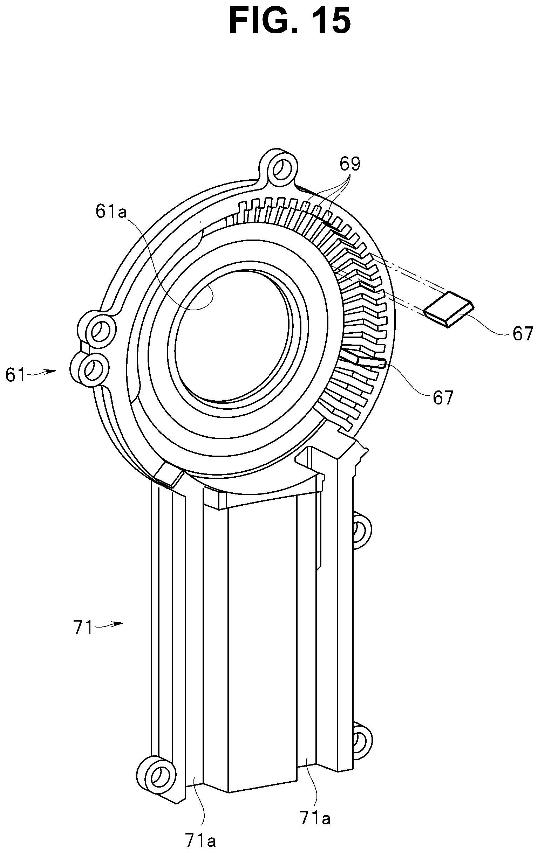

Fig. 15 is a perspective view showing a first case member enlarged.
Fig. 16 is a side view showing the vertical bending pulley and the lateral bending pulley.
Fig. 17 is a sectional view taken along the line XVII-XVII in Fig. 16.
Fig. 18 is a sectional view taken along the line XVIII-XVIII in Fig. 16.
Fig. 19 is a sectional view taken along the line XIX-XIX in Fig. 16.
Fig. 20 is a sectional view taken along the line XX-XX in Fig. 16.
Fig. 21 is a plan view schematically showing a state where the vertical bending pulley is rotated to a first rotation end position.
Fig. 22 is a plan view schematically showing a state where the vertical bending pulley is rotated to a second rotation end position.
Fig. 23 is an exploded perspective view showing a lateral bending operation knob from one end side.
Fig. 24 is an exploded perspective view showing the lateral bending operation knob from another end side.
Fig. 25 is an exploded perspective view showing, in an enlarged manner, a main part of the lateral bending operation knob from the one end side.
Fig. 26 is an exploded perspective view showing, in an enlarged manner, the main part of the lateral bending operation knob from the other end side.
Fig. 27 is a perspective view showing a cam plate enlarged.
Fig. 28 is a sectional view of a main part of the lateral bending operation knob when a break is released.
Fig. 29 is a sectional view of the main part of the lateral bending operation knob when the break is operated.
Fig. 30 is an exploded perspective view showing a vertical bending operation knob from one end side.
Fig. 31 is an exploded perspective view showing the vertical bending operation knob from another end side.
Fig. 32 is a sectional view of a main part of the vertical bending operation knob when the break is released.
Fig. 33 is a sectional view of the main part of the vertical bending operation knob when the break is operated.
Fig. 34 is a perspective view showing a pulley unit from one end side.
Fig. 35 is a perspective view showing a raising base (forceps elevator) operation mechanism.
Fig. 36 is a sectional view of a main part of a cylinder unit.
Fig. 37 is a perspective view showing a raising base operation lever attached to the operation portion.
Fig. 38 is an exploded perspective view showing the raising base operation lever and angle adjustment plates.
Fig. 39 is an explanatory diagram showing a rotation angle range of the raising base operation lever when the angle adjustment plate is not attached.
Fig. 40 is an explanatory diagram showing the rotation angle range of the raising base operation lever when the angle adjustment plate is not attached.
Fig. 41 is an explanatory diagram showing the rotation angle range of the raising base operation lever when the angle adjustment plate is attached.
Fig. 42 is an explanatory diagram showing the rotation angle range of the raising base operation lever when the angle adjustment plate is attached.

### Modes for Carrying Out the Invention

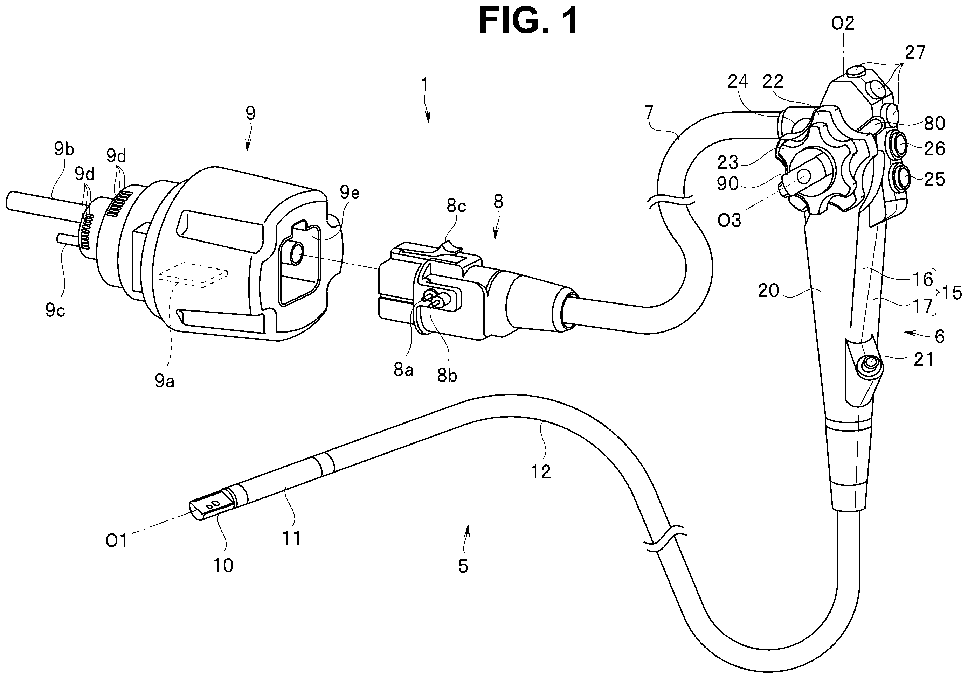

Hereinafter, forms of the present invention will be described with reference to drawings. The drawings are according to one embodiment of the present invention, and Fig. 1 is a perspective view showing an endoscope. An endoscope 1 shown in Fig. 1 is a medical single-use endoscope that is used only once, for example. The endoscope 1 includes an insertion portion 5, an operation portion 6, a universal cord 7, and an endoscope connector 8.

The insertion portion 5 includes a distal end portion 10, a bending portion 11, and a flexible tube portion 12 in this order from a distal end side of the insertion portion 5.

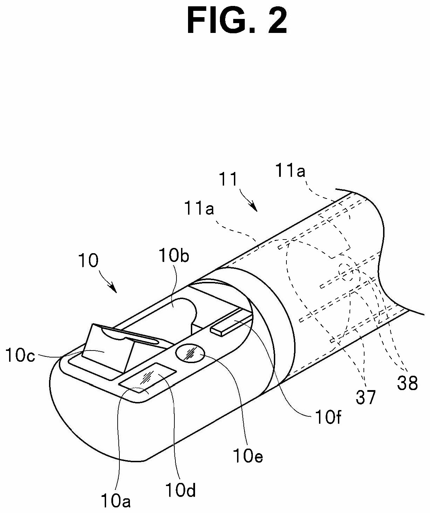

As shown in Fig. 2, the distal end portion 10 is formed of a rigid member and has a substantially cylindrical shape. The distal end portion 10 includes a flat portion 10a, an opening portion 10b, and a raising base 10c.

The flat portion 10a is formed on a part of an outer circumference side portion of the distal end portion 10. The flat portion 10a is provided with an illumination window 10d, an observation window 10e, and a nozzle 10f.

The illumination window 10d is formed of an optical member located at a distal-most end of an illumination optical system. The illumination optical system can irradiate a subject with an illumination light guided from a light source by a light guide or the like, for example.

The observation window 10e is formed of an observation optical system of an image pickup unit provided in the distal end portion 10. That is, the observation window 10e is formed by exposing an optical member located at a distal-most end of the observation optical system from the flat portion 10a. The observation optical system takes in a return light from the subject from the observation window 10e. Then, the observation optical system projects the return light that is taken in to an image pickup device of the image pickup unit. Therefore, the image pickup device can pick up a subject image by converting the return light into an image pickup signal. Here, an optical axis of the observation optical system is set in a direction intersecting a longitudinal axis O1 of the insertion portion 5 (lateral view direction).

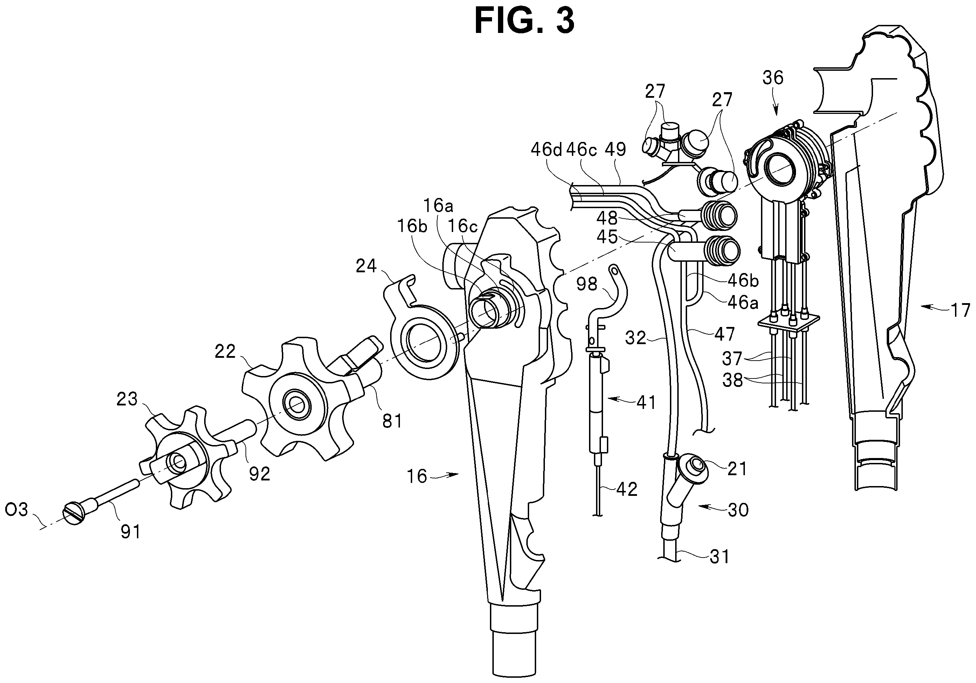

The nozzle 10f is connected to an end portion on a distal end side of a gas/liquid feeding tube 47 (see Fig. 3) described later. Therefore, the nozzle 10f can discharge a gas or a liquid supplied from the gas/liquid feeding tube 47 to the flat portion 10a.

The opening portion 10b is formed to open in a side portion of the distal end portion 10 in an outer circumference direction. The opening portion 10b is communicated with a treatment instrument channel 31 described later.

The raising base 10c is a member for raising a distal end side of a treatment instrument that protrudes from the treatment instrument channel 31. Therefore, the raising base 10c is disposed at a position facing a distal end of the treatment instrument channel 31 in the opening portion 10b. In addition, the raising base 10c is swingably attached to the distal end portion 10. Furthermore, the raising base 10c is connected to a distal end side of a raising base operation wire 42 (see Fig. 3) described later.

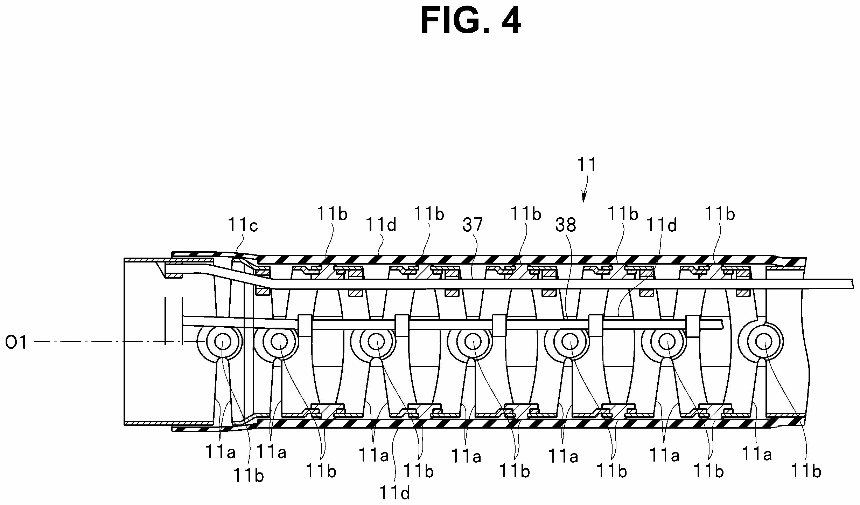

The bending portion 11 includes a plurality of bending pieces 11a (see Figs. 2 and 4). The bending pieces 11a are arranged in a row along the longitudinal axis O1 of the insertion portion 5. Furthermore, the bending pieces 11a adjacent to each other of the bending pieces 11a are rotatably coupled to each other via a coupling member 11b such as a rivet. Therefore, the plurality of bending pieces 11a configure a bending piece string which allows bending in all directions including up, down, left, and right directions.

Note that in the present embodiment, the up, down, left, and right directions of the insertion portion 5 are directions that intersect, for example, the longitudinal axis O1 and are defined as corresponding to up, down, left, and right directions of an image picked up by the image pickup device in the distal end portion 10.

The bending piece 11a located at a distal-most end of the bending portion 11 is connected to distal end portions of a pair of the vertical bending operation wires 37 and distal end portions of a pair of the lateral bending operation wires 38 (in Fig. 4, only the vertical bending operation wire 37 that pulls the bending portion 11 in the up direction and the lateral bending operation wire 38 that pulls the bending portion 11 in the left direction are shown).

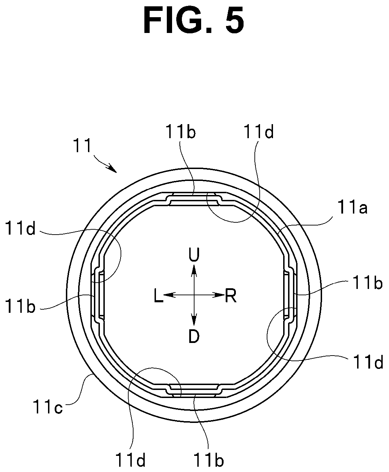

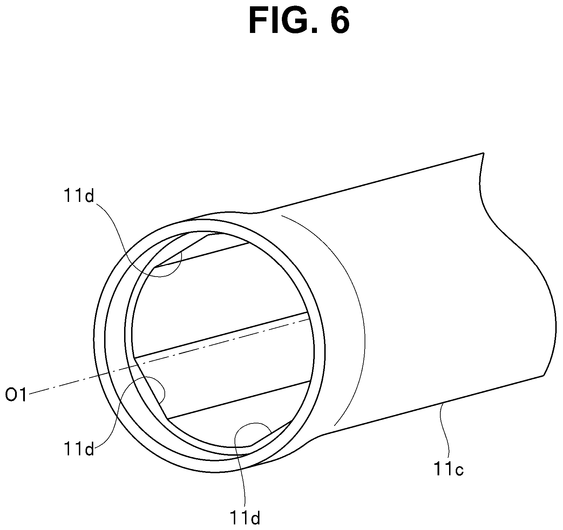

Furthermore, the outer circumference of the bending piece string is covered by an outer sheath 11c. The outer sheath 11c is formed of, for example, a rubber member having a substantially cylindrical shape. A thickness of the outer sheath 11c is set to 0.5 mm, for example. However, as shown in Fig. 4 to Fig. 6, in an inner circumference side of the outer sheath 11c, thickened portions 11d are formed respectively at positions corresponding to up, down, left, and right bending directions. A thickness of the thickest part of each of the thickened portions 11d is set to 0.7 mm, for example. Each of the thickened portions 11d extends in the direction of the longitudinal axis O1 of the bending portion 11 in a state where the thickened portions 11d abut on the coupling members 11b respectively.

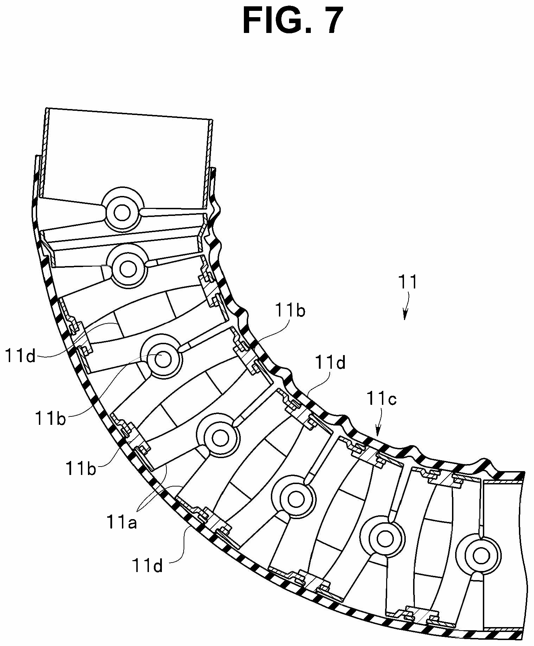

Each of the thickened portions 11d partially increases an elastic force of the outer sheath 11c. Therefore, when the bending portion 11 is bent, each of the thickened portions 11d prevents a part of the outer sheath 11c from elastically deforming toward an inside of the bending portion 11. More specifically, for example, as shown in Fig. 7, when the bending portion 11 is bent, the thickened portion 11d located on an inner side of the bending portion 11 in the bending direction causes a part of the outer sheath 11c to elastically deform toward an outside of the bending portion 11.

With such elastic deformation, when the bending portion 11 is bent, each of the thickened portions 11d prevents the part of the outer sheath 11c from being caught between the adjacent bending pieces 11a to be damaged. Therefore, for example, in a single-use endoscope which is expected to be used only in a short time period, the bending portion 11 can be configured without disposing a braid or the like between respective the bending pieces 11a (bending piece string) and the outer sheath 11c.

The flexible tube portion 12 is a tube portion that can bend according to a shape of the subject into which the insertion portion 5 is inserted. Here, in the present embodiment, a flexible endoscope including the flexible tube portion 12 is described as an example of the endoscope 1, but the endoscope 1 may be a rigid endoscope including a rigid tube portion.

The operation portion 6 includes a housing 15 as an operation portion main body. As shown in Figs. 1 and 3, the housing 15 is divided to the left and right and is formed, for example, by a first housing member 16 and a second housing member 17. The first and second housing members 16 and 17 are, for example, formed by a resin molding. The first housing member 16 and the second housing member 17 are bonded together using an adhesive or the like, to form the hollow housing 15.

As shown in Fig. 4, a proximal end side of the flexible tube portion 12 is connected to a distal end side of the housing 15.

The housing 15 includes a grasping portion 20 that is for an operator to grasp the operation portion 6 with a hand and that is formed at a substantially central portion in a longitudinal axis O2 direction of the housing 15. At a position on a distal end side with respect to the grasping portion 20, the housing 15 includes a channel port 21 for inserting the treatment instrument attached. At a position on a proximal end side with respect to the grasping portion 20, the housing 15 is provided with a vertical bending operation knob 22 and a lateral bending operation knob 23 as bending operation members, a raising base operation lever 24, a gas/liquid feeding button 25, a suction button 26, and a plurality of button switches 27.

As shown in Figs. 1 and 3, the channel port 21 is held by the housing 15 in a state sandwiched between the first housing member 16 and the second housing member 17. The channel port 21 in the present embodiment is formed in a part of a branch tube 30 disposed inside the housing 15. Specifically, the branch tube 30 in the present embodiment includes a branch conduit that branches off laterally from a middle of the conduit that extends in a vertical direction. Such branch tube 30 is, for example, integrally molded using a resin. The channel port 21 is formed on an end portion of the branch conduit. Note that the branch tube 30 may be made of metal.

A proximal end side of the treatment instrument channel 31 that is inserted through the insertion portion 5 is connected to an end portion of a distal end side of the branch tube 30. Therefore, the channel port 21 is communicated with the opening portion 10b of the distal end portion 10 through the treatment instrument channel 31.

A distal end side of a first suction tube 32 is connected to an end portion of a proximal end side of the branch tube 30. The first suction tube 32 is communicated with the opening portion 10b of the distal end portion 10 through the treatment instrument channel 31.

The vertical bending operation knob 22 and the lateral bending operation knob 23 are rotatably attached to a side portion of the first housing member 16 in a state overlapped on the same central axis O3. The vertical bending operation knob 22 and the lateral bending operation knob 23 configure an endoscope bending operation mechanism (hereinafter, referred to as a bending operation mechanism) 35, together with a pulley unit 36 described later. The bending operation mechanism 35 pulls or relaxes the vertical bending operation wire 37 and the lateral bending operation wire 38 according to operation amounts to the vertical bending operation knob 22 and the lateral bending operation knob 23. Therefore, the bending operation mechanism 35 can bend the bending portion 11 in the all direction including the up, down, left, and right directions.

The raising base operation lever 24 is rotatably attached to the side portion of the first housing member 16 between the vertical bending operation knob 22 and the first housing member 16. The raising base operation lever 24 configures a raising base operation mechanism 40 together with a cylinder unit 41 described later. The raising base operation mechanism 40 pulls or relaxes the raising base operation wire 42 according to an operation amount to the raising base operation lever 24. Therefore, the raising base operation mechanism 40 can swing the raising base 10c.

The gas/liquid feeding button 25 is an operation button for feeding a gas or a liquid from the nozzle 10f to the flat portion 10a of the distal end portion 10. The gas/liquid feeding button 25 is attached to the housing 15 through a gas/liquid feeding cylinder 45.

As shown in Fig. 3, a proximal-end-side end portion of a first gas feeding tube 46a, a proximal-end-side end portion of a first liquid feeding tube 46b, a distal end side end portion of a second gas feeding tube 46c, and a proximal-end-side end portion of a second liquid feeding tube 46d are connected to the gas/liquid feeding cylinder 45. Here, both of a distal end side end portion of the first gas feeding tube 46a and a distal end side end portion of the first liquid feeding tube 46b are connected to a proximal-end-side end portion of the gas/liquid feeding tube 47.

Inside the gas/liquid feeding cylinder 45, a not-shown piston that is connected to the gas/liquid feeding button 25 is provided. The piston advances and retreats inside the gas/liquid feeding cylinder 45 according to a pressing state to the gas/liquid feeding button 25. According to an advance/retreat position in the gas/liquid feeding cylinder 45, the piston communicates or blocks between the first gas feeding tube 46a and the second gas feeding tube 46c, and communicates or blocks between the first liquid feeding tube 46b and the second liquid feeding tube 46d.

The suction button 26 is an operation button to suction a liquid or a solid matter through the opening portion 10b provided in the distal end portion 10. The suction button 26 is attached to the housing 15 through a suction cylinder 48.

As shown in Fig. 3, a proximal end side of the first suction tube 32 and a distal end side of a second suction tube 49 are connected to the suction cylinder 48.

The suction cylinder 48 includes inside thereof a not-shown piston. The piston advances or retreats inside the suction cylinder 48 according to a pressing state to the suction button 26. The piston communicates or blocks between the first suction tube 32 and the second suction tube 49 according to an advance/retreat position in the suction cylinder 48.

The plurality of button switches 27 are held by the housing 15 in a state sandwiched between the first housing member 16 and the second housing member 17. The button switches 27 can be allocated as respective switches for operating various functions of the endoscope 1.

The universal cord 7 is extended from a proximal end side of the operation portion 6. Tubes such as the second gas feeding tube 46c, the second liquid feeding tube 46d, and the second suction tube 49 are inserted through an inside of the universal cord 7. Also, various signal cables connected to each of the button switches and the image pickup device of the image pickup unit and the like are inserted through the inside of the universal cord 7. Furthermore, a light guide bundle and the like, which are optically connected to the illumination optical system, are inserted through the inside of the universal cord 7.

The endoscope connector 8 is connected to an extended end portion of the universal cord 7. The endoscope connector 8 can be connected to external devices (not shown) such as a light source apparatus and a processor through a relay connector 9.

As shown in Fig. 1, the endoscope connector 8 in the present embodiment has a substantially quadrangular prism shape, for example. The endoscope connector 8 includes, on a side surface thereof, for example, a liquid feeding connector 8a connected to the second liquid feeding tube 46d, and a gas feeding connector 8b connected to the second gas feeding tube 46c. The endoscope connector 8 also includes, on a side surface thereof, an engaging pawl 8c that can be engaged with the relay connector 9. Furthermore, the endoscope connector 8 includes, on an end surface thereof, a suction connector connected to the second suction tube 49, a plurality of electric connectors connected to various signal cables, and a light guide connector connected to the light guide bundle (none of these are shown). Note that the suction connector is disposed on a side surface opposite to the side surface, where the liquid feeding connector 8a and the gas feeding connector 8b are located, of the side surfaces of the endoscope connector 8.

The relay connector 9 is a reusable product that can be used a plurality of times. That is, the relay connector 9 can be used repeatedly for a plurality of endoscopes 1 (single-use endoscopes). The relay connector 9 has a substantially columnar shape, for example.

The relay connector 9 includes a control substrate 9a inside thereof. The control substrate 9a performs various signal processing on an image pickup signal, and correction processing of a power supply current supplied to the endoscope 1, or other processing, for example.

The relay connector 9 includes a light source connector 9b and a gas feeding plug 9c on a distal end surface thereof. The relay connector 9 also includes a plurality of electric contact points 9d on side surfaces thereof.

Furthermore, the relay connector 9 includes, in a proximal end portion, a connector receiving hole 9e into which the endoscope connector 8 can be inserted. The connector receiving hole 9e has, for example, a substantially quadrangular hole shape. Inside the connector receiving hole 9e, a proximal end side of the light source connector 9b is protruded. A proximal end side of the light source connector 9b is located at a position that can be optically connected to the light guide connector of the endoscope connector 8. Inside the connector receiving hole 9e, a gas feeding cap that is connected to the gas feeding plug 9c, and an electric connector receiver that is connected to each of the electric contact points 9d are provided (none of them are shown). Among those, the gas feeding cap is provided at a position that can be connected to the gas feeding plug of the endoscope connector 8. The electric connector receiver is provided at a position that can be connected to the electric connector of the endoscope connector 8.

Next, a configuration of the bending operation mechanism 35 will be described in detail.

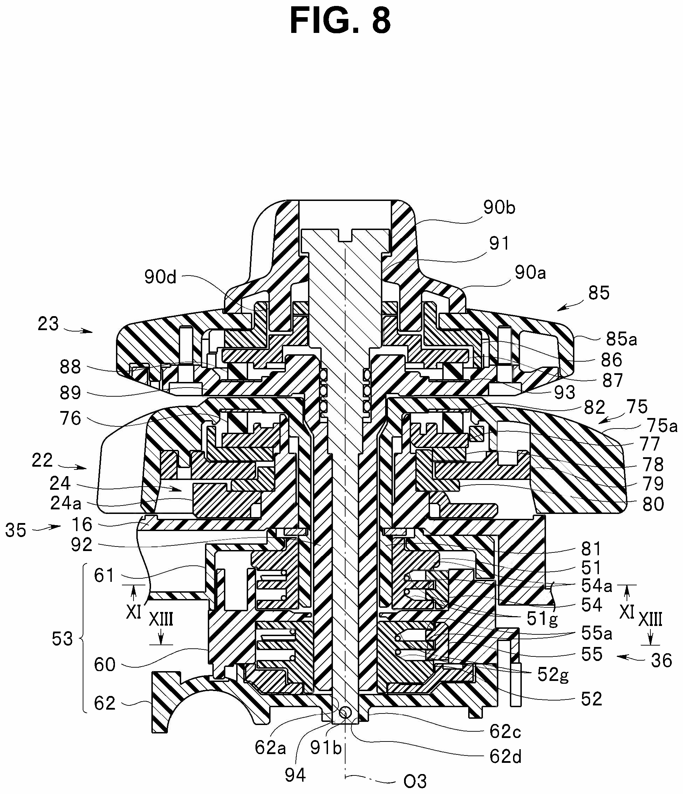

As shown in Fig. 8 to Fig. 10, the pulley unit 36 of the bending operation mechanism 35 includes a vertical bending pulley 51, a lateral bending pulley 52, and a pulley case 53. Note that in the following description of the bending operation mechanism 35, a side from the vertical bending pulley 51 toward the lateral bending pulley 52 along the central axis O3 direction of the bending operation mechanism 35 is referred to as one end (one side), and a side from the lateral bending pulley 52 toward the vertical bending pulley 51 along the central axis O3 direction of the bending operation mechanism 35 is referred to as the other end (the other side).

The vertical bending pulley 51 includes a vertical bending pulley main body 51a that has a predetermined thickness and has a substantially disk shape.

The vertical bending pulley main body 51a includes a key hole 51b that penetrates through the central portion thereof in the central axis O3 direction.

The vertical bending pulley main body 51a includes a pair of pulley grooves 51c as grooves on an outer circumference portion thereof.

The vertical bending pulley main body 51a includes wire stopper receiving holes 51d on both respective side portions thereof (both respective side portions of the vertical bending pulley main body 51a in the central axis O3 direction). Furthermore, the vertical bending pulley main body 51a includes, on the both respective side portions thereof, connection grooves 51e for connecting the wire stopper receiving holes 51d to the pulley grooves 51c, respectively.

The vertical bending pulley main body 51a includes a convex portion 51f on another side portion thereof. In the present embodiment, the convex portion 51f has a partially arc shape, for example, and protrudes in a radially outward direction of the vertical bending pulley main body 51a.

The vertical bending pulley 51 configured in this way allows proximal end sides of the pair of the vertical bending operation wires 37 to be wound inside of the pulley grooves 51c respectively. In this case, each of the wire stopper receiving holes 51d is inserted with a wire stopper 37a attached to the proximal end portion of each of the vertical bending operation wires 37. Therefore, each of the vertical bending operation wires 37 is coupled to the vertical bending pulley 51. Then, the vertical bending operation wires 37 are guided to the pulley grooves 51c respectively via the connection grooves 51e. Therefore, the vertical bending operation wires 37 are wound in the pulley grooves 51c respectively, with a position where each of the wire stopper receiving holes 51d and each of the connection grooves 51e are provided, as a starting position for winding.

The pair of the vertical bending operation wires 37 are wound in directions opposite to each other in a circumference direction of the pair of the pulley grooves 51c. Therefore, with the rotation of the vertical bending pulley 51 around the central axis O3, when one of the vertical bending operation wires 37 is pulled, the other of the vertical bending operation wires 37 is relaxed. When the one of the vertical bending operation wires 37 is relaxed, the other of the vertical bending operation wires 37 is pulled.

Here, a diameter of each of the vertical bending operation wires 37 may be 0.25 mm or more and 0.55 mm or less, or may be 0.4 mm or more and 0.55 mm or less.

Note that in the following description, when distinguishing between the vertical bending operation wire 37 and the pulley groove 51c for bending the bending portion 11 upward, and the vertical bending operation wire 37 and the pulley groove 51c for bending the bending portion 11 downward, etc., among the pair of the vertical bending operation wires 37 and the pair of the pulley grooves 51c, the letter "u" or "d" is added to the end of the reference signs, as appropriate.

Here, a bottom portion of each of the pulley grooves 51c has a width in which at most two vertical bending operation wires 37 can be disposed side by side in a rotation axis direction of the vertical bending pulley 51 (central axis O3 direction), for example. That is, each of the pulley grooves 51c has a groove width that allows each of the vertical bending operation wires 37 to be wound along the bottom portion of each of the pulley grooves 51c for at most two rounds. In other words, the pulley grooves 51c are configured by parallel grooves each having a groove width which is twice or more of the diameter of the vertical bending operation wire 37, for example. Note that, in addition, each of the pulley grooves 51c may have a groove width which is less than three times the diameter of the vertical bending operation wire 37.

Furthermore, each of the pulley grooves 51c includes a wire guide 51g inside thereof. Each of the wire guides 51g guides each of the vertical bending operation wires 37 to be wound in each of the pulley grooves 51c to a predetermined winding position.

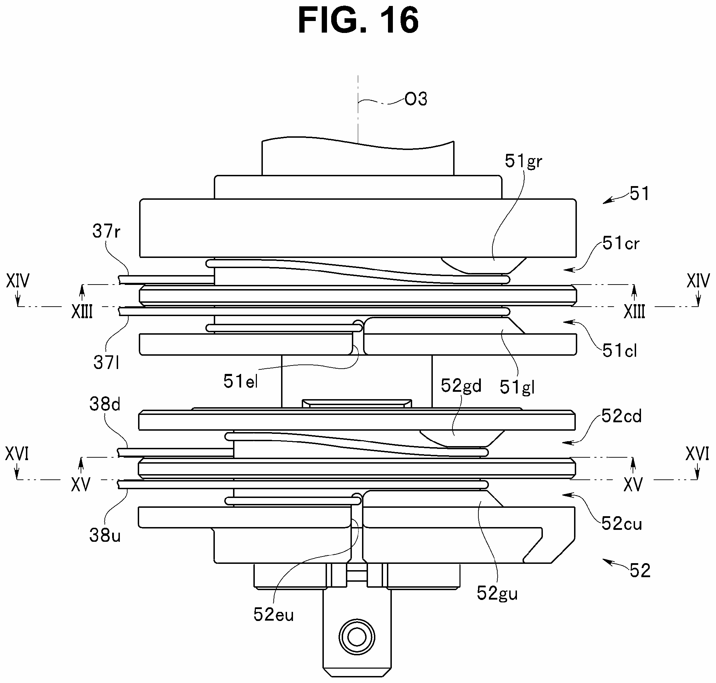

As shown in Figs. 8 and 16 to 18, each of the wire guides 51g is configured by a tapered projection provided in a partial region of a circumference direction of each of the pulley grooves 51c.

The projection configuring each of the wire guides 51g extends from a position adjacent to each of the connection grooves 51e in a direction opposite to a winding direction of each of the vertical bending operation wires 37. As a more specific example, each of the wire guides 51g may be provided in a region that occupies approximately 1/4 of the entire circumference of each of the pulley grooves 51c.

A width of the bottom portion of each of the pulley grooves 51c in the partial region of each of the pulley grooves 51c is narrowed down with each of such wire guides 51g. Specifically, the width of the bottom portion in the partial region of each of the pulley grooves 51c is narrowed down with each of the wire guides 51g, to a width in which only one vertical bending operation wire 37 can be disposed in the central axis O3 direction. That is, the width of the bottom portion in the partial region of each of the pulley grooves 51c is narrowed down with each of the wire guides 51g so that the vertical bending operation wire 37 can be wound only one round in the central axis O3 direction. In other words, the width of the bottom portion of each of the pulley grooves 51c is narrowed down with each of the wire guides 51g, to a groove width that is equal to or more than the diameter of the vertical bending operation wire 37, and is less than twice of the diameter of the vertical bending operation wire 37.

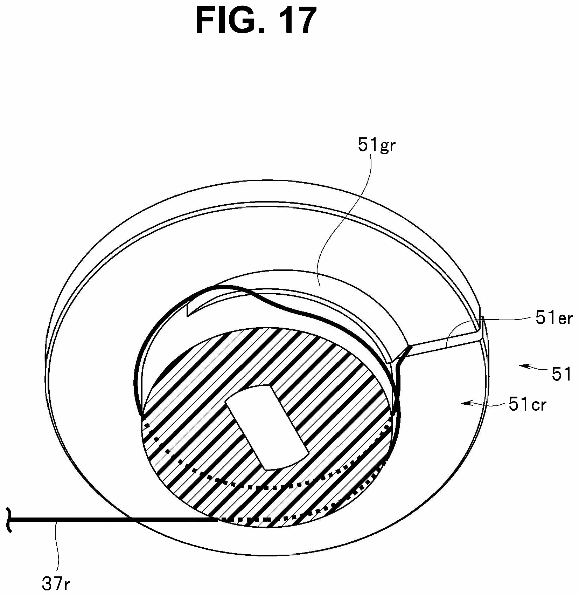

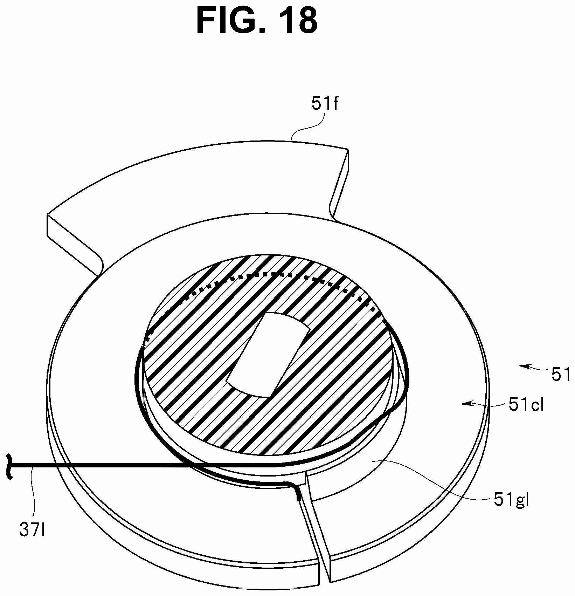

Furthermore, each of the wire guides 51g guides each of the vertical bending operation wires 37 to be wound in the partial region of each of the pulley grooves 51c to the bottom portion of each of the pulley grooves 51c with the narrowed groove width. That is, each of the wire guides 51g guides each of the vertical bending operation wires 37 to a bottom surface along each of the tapered surfaces. With this, each of the wire guides 51g controls a winding position of each of the vertical bending operation wires 37 with respect to each of the pulley grooves 51c when a winding state of each of the vertical bending operation wires 37 with respect to each of the pulley grooves 51c changes from a first round to a second round. Note that for ease of understanding, a thickness of each of the vertical bending operation wires 37 in Figs. 17 and 18 is shown as being thinner than a thickness obtained from a dimension ratio with the groove width of each of the pulley grooves 51c.

As above, in the present embodiment, the vertical bending pulley 51 corresponds to a specific example of a first pulley. In addition, the pulley groove 51cu corresponds to a specific example of a first groove, and the pulley groove 51cd corresponds to a specific example of a second groove. In addition, the vertical bending operation wire 37u corresponds to a specific example of a first wire, and the vertical bending operation wire 37d corresponds to a specific example of a second wire. Furthermore, the wire guide 51gu corresponds to a specific example of a first wire guide, and the wire guide 51gd corresponds to a specific example of a second wire guide.

The vertical bending pulley 51 includes a protective member 54 as a cover mounted on a part of the outer circumference thereof. The protective member 54 has a shape in which a part of an annular shape is cut out. That is, the protective member 54 has an arc shape. In this case, a central angle of the arc of the protective member 54 is set to be more than 180 degrees. Therefore, the protective member 54 has a substantially C-shape. An inner surface of the protective member 54 formed in this way has an arc shape.

The protective member 54 covers a part of each of the pulley grooves 51c from an outer circumference side of the vertical bending pulley 51. Therefore, the inner surface of the protective member 54 is arranged on the same axis as the rotation axis of the vertical bending pulley 51 (central axis O3).

Furthermore, the inner surface of the protective member 54 is provided with projections 54a each protruding toward the inside of each of the pulley grooves 51c. The projections 54a are disposed on the inner surface of the protective member 54 in a state of being adjacent to each other. In addition, each of the projection 54a extends in the circumference direction along the inner surface of the protective member 54. Therefore, an inner surface shape (tip shape) of each of the projections 54a has an arc shape. In this case, a central angle of the arc of each of the projections 54a is set to be more than 180 degrees. That is, each of the projections 54a has a substantially C-shape.

Note that in the following description, when distinguishing between the projection 54a corresponding to the pulley groove 51cu and the projection 54a corresponding to the pulley groove 51cd of the pair of the projections 54a, the letter "u" or "d" is added to the end of the reference signs, as appropriate.

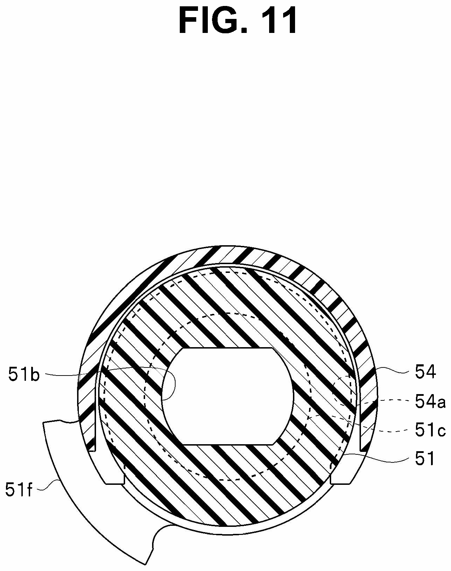

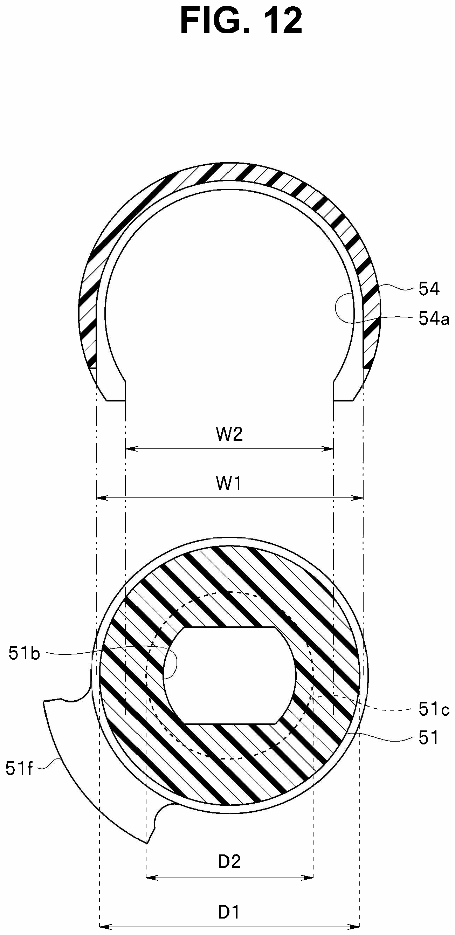

Here, for example, as shown in Fig. 12, a distance W1 between both end portions of the protective member 54 is set to be equal to or more than a diameter D1 of the vertical bending pulley 51. A distance W2 between both end portions of each of the projections 54a is set to be sufficiently greater than a diameter D2 of each of the pulley grooves 51c. Therefore, the protective member 54 can be attached to the vertical bending pulley 51 only by inserting the projections 54a each having the arc shape into an outer circumference side of the pulley grooves 51c, respectively (see Fig. 11). That is, the protective member 54 can be inserted into the outer circumference side of the vertical bending pulley 51 without deforming the substantially C-shape of the protective member 54. Similarly, the projections 54a can be inserted into the outer circumference side of the pulley grooves 51c, respectively, without deforming the substantially C-shape of the projections 54a. Note that as another example, the protective member 54 and the projections 54a can be integrally formed by a material having elasticity. The protective member 54 and the projections 54a thus can be elastically deformed in a direction in which the both end portions in the circumference direction away from each other. With this, for example, even in a case where the distance W1 between the both end portions of the protective member 54 is set to be less than the diameter D1 of the vertical bending pulley 51, the distance W1 can be temporarily changed to be equal to or more than the diameter D1 by the elastic deformation of the protective member 54. Therefore, the protective member 54 and the projections 54a can be mounted to the vertical bending pulley 51 and the pulley grooves 51c respectively from the outer circumference sides of the vertical bending pulley 51 and the pulley grooves 51c.

As shown in Fig. 8, the projections 54a include shapes that fit into the pulley grooves 51c respectively when the protective member 54 is mounted to the vertical bending pulley 51. Therefore, the projections 54a are located on the same axis as the central axis O3 of the vertical bending pulley 51.

As shown in Figs. 8 to 10, the both end portions of the protective member 54 and the both end portions of each of the projections 54a in the circumference direction are located in a direction in which each of the vertical bending operation wires 37 extends from each of the pulley grooves 51c of the vertical bending pulley 51. Therefore, advance/retreat movement of each of the vertical bending operation wires 37 with rotation of the vertical bending pulley 51 is allowed.

In addition, the projections 54a that fit into the pulley grooves 51c respectively restrict the movement of the vertical bending operation wire 37 in the radially outward direction of the vertical bending pulley 51. Therefore, the protective member 54 prevents the vertical bending operation wire 37 from falling off the pulley groove 51c when being relaxed.

As above, in the present embodiment, the protective member 54 corresponds to a specific example of a cover. In addition, the projection 54au corresponds to a specific example of a first projection, and the projection 54ad corresponds to a specific example of a second projection.

The lateral bending pulley 52 includes a lateral bending pulley main body 52a that has a predetermined thickness and has a substantially disk shape.

The lateral bending pulley main body 52a includes at the central portion thereof a key hole 52b that penetrates in the central axis O3 direction.

The lateral bending pulley main body 52a includes a pair of pulley grooves 52c as grooves on an outer circumference portion thereof.

The lateral bending pulley main body 52a includes wire stopper receiving holes 52d on both respective side portions thereof. Furthermore, the lateral bending pulley main body 52a includes, on the both respective side portions thereof, connection grooves 52e for connecting the wire stopper receiving holes 52d to the pulley grooves 52c respectively.

The lateral bending pulley main body 52a includes a convex portion 52f on one side portion thereof. In the present embodiment, the convex portion 52f has a partial arc shape, for example, and protrudes in a radially outward direction of the lateral bending pulley main body 52a.

The lateral bending pulley 52 configured in this way allows proximal end sides of the pair of the lateral bending operation wires 38 to be wound inside of the pulley grooves 52c respectively. In this case, a wire stopper 38a attached to the proximal end portion of each of the lateral bending operation wires 38 is inserted into each of the wire stopper receiving holes 52d. Therefore, each of the lateral bending operation wires 38 is coupled to the lateral bending pulley 52. Then, the lateral bending operation wires 38 are guided to the pulley grooves 52c respectively via each of the connection grooves 52e.

The pair of the lateral bending operation wires 38 are wound in directions opposite to each other in a circumference direction of the pair of the pulley grooves 52c. Therefore, with rotation of the lateral bending pulley 52 around the central axis O3, when one of the lateral bending operation wires 38 is pulled, the other of the lateral bending operation wires 38 is relaxed. When the one of the lateral bending operation wires 38 is relaxed, the other of the lateral bending operation wires 38 is pulled.

Note that in the following description, when distinguishing between the lateral bending operation wire 38 and the pulley groove 52c for bending the bending portion 11 leftward, and the lateral bending operation wire 38 and the pulley groove 52c for bending the bending portion 11 rightward, among the pair of the lateral bending operation wires 38 and the pair of the pulley grooves 52c, the letter "l" or "r" is added to the end of the reference signs, as appropriate.

Here, a bottom portion of each of the pulley grooves 52c has a width in which at most two lateral bending operation wires 38 can be disposed side by side in a rotation axis direction of the lateral bending pulley 52 (central axis O3 direction), for example. That is, each of the pulley grooves 52c has a groove width that allows each of the lateral bending operation wires 38 to be wound along the bottom portion of each of the pulley grooves 52c for at most two rounds. In other words, the pulley grooves 52c are configured by parallel grooves each having a groove width which is twice or more of the diameter of the lateral bending operation wire 38, for example. Note that, in addition, each of the pulley grooves 52c may have a groove width which is less than three times the diameter of the lateral bending operation wire 38.

Furthermore, each of the pulley grooves 52c includes a wire guide 52g inside thereof. Each of the wire guides 52g guides each of the lateral bending operation wires 38 to be wound in each of the pulley grooves 52c to a predetermined winding position.

As shown in Figs. 8, 16, 19, and 20, each of the wire guides 52g is configured by a tapered projection provided in a partial region of a circumference direction of each of the pulley grooves 52c.

The projection configuring each of the wire guides 52g extends from a position adjacent to each of the connection grooves 52e in a direction opposite to a winding direction of each of the lateral bending operation wires 38. As a more specific example, each of the wire guides 52g may be provided in a region that occupies approximately 1/4 of the entire circumference of each of the pulley grooves 52c.

A width of the bottom portion of each of the pulley grooves 52c in the partial region of each of the pulley grooves 52c is narrowed down with each of such wire guides 52g. Specifically, the width of the bottom portion of each of the pulley grooves 52c is narrowed down with each of the wire guides 52g, to a width in which only one lateral bending operation wire 38 can be disposed in the central axis O3 direction. That is, the width of the bottom portion in the partial region of each of the pulley grooves 52c is narrowed down with each of the wire guides 52g so that the lateral bending operation wire 38 can be wound only one round in the central axis O3 direction. In other words, the width of the bottom portion of each of the pulley grooves 52c is narrowed down with each of the wire guides 52g, to a groove width that is equal to or more than the diameter of the lateral bending operation wire 38, and is less than twice of the diameter of the lateral bending operation wire 38.

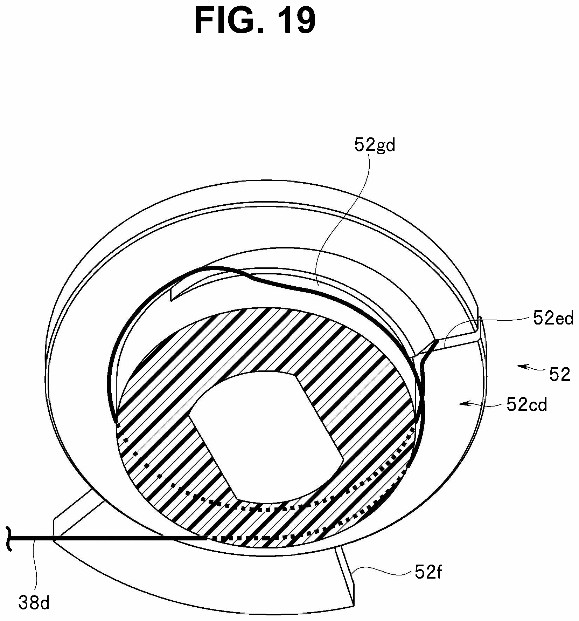

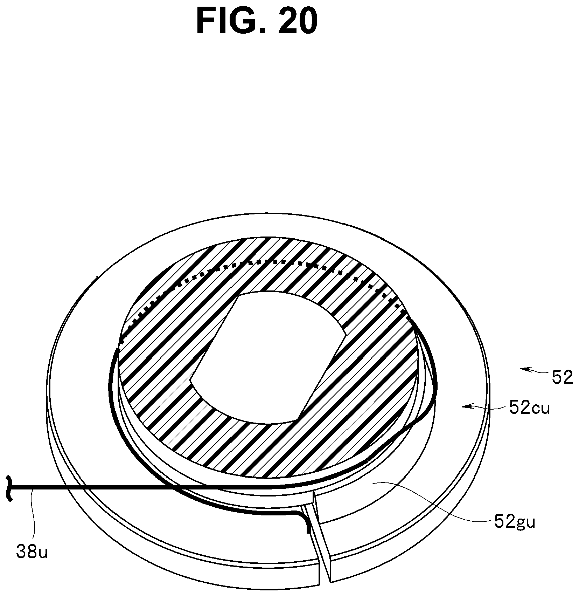

Furthermore, each of the wire guides 52g guides each of the lateral bending operation wires 38 to be wound in the partial region of each of the pulley groove 52c to the bottom portion of each of the pulley grooves 52c with the narrowed groove width. That is, each of the wire guides 52g guides each of the lateral bending operation wires 38 to a bottom surface along each of the tapered surfaces. With this, each of the wire guides 52g controls a winding position of each of the lateral bending operation wires 38 with respect to each of the pulley grooves 52c when a winding state of each of the lateral bending operation wires 38 with respect to each of the pulley grooves 52c changes from a first round to a second round. Note that for ease of understanding, a thickness of each of the lateral bending operation wires 38 in Figs. 19 and 20 is shown as being thinner than a thickness obtained from a dimension ratio with the groove width of each of the pulley grooves 52c.

As above, in the present embodiment, the lateral bending pulley 52 corresponds to a specific example of a second pulley. In addition, the pulley groove 52cu corresponds to a specific example of a third groove, and the pulley groove 52cd corresponds to a specific example of a fourth groove. In addition, the lateral bending operation wire 38u corresponds to a specific example of a third wire, and the lateral bending operation wire 38d corresponds to a specific example of a fourth wire. Furthermore, the wire guide 52gu corresponds to a specific example of a third wire guide, and the wire guide 52gd corresponds to a specific example of a fourth wire guide.

The lateral bending pulley 52 includes a protective member 55 as a cover mounted on a part of the outer circumference thereof. The protective member 55 has a shape in which a part of an annular shape is cut out. That is, the protective member 55 has an arc shape. In this case, a central angle of the arc of the protective member 55 is set to be more than 180 degrees. Therefore, the protective member 55 has a substantially C-shape. An inner surface of the protective member 55 formed in this way has an arc shape.

Furthermore, the inner surface of the protective member 55 is provided with projections 55a each protruding toward the inside of each of the pulley grooves 52c. An inner surface shape of each of the projections 55a has an arc shape.

The protective member 55 covers a part of each of the pulley grooves 52c from an outer circumference side of the lateral bending pulley 52. Therefore, the inner surface of the protective member 55 is arranged on the same axis as the rotation axis of the lateral bending pulley 52 (central axis O3).

Furthermore, the projections 55a are disposed on the inner surface of the protective member 55 in a state of being adjacent to each other. In addition, each of the projections 55a extends in the circumference direction along the inner surface of the protective member 55. Therefore, an inner surface shape (tip shape) of each of the projections 55a has an arc shape. In this case, a central angle of the arc of each of the projections 55a is set to be more than 180 degrees. That is, each of the projections 55a has a substantially C-shape.

Note that in the following description, when distinguishing between the projection 55a corresponding to the pulley groove 52cl and the projection 55a corresponding to the pulley groove 52cr of the pair of the projections 55a, the letter "l" or "r" is added to the end of the reference signs, as appropriate.

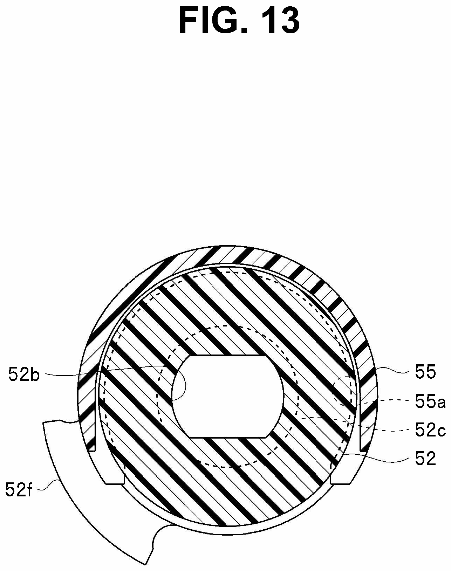

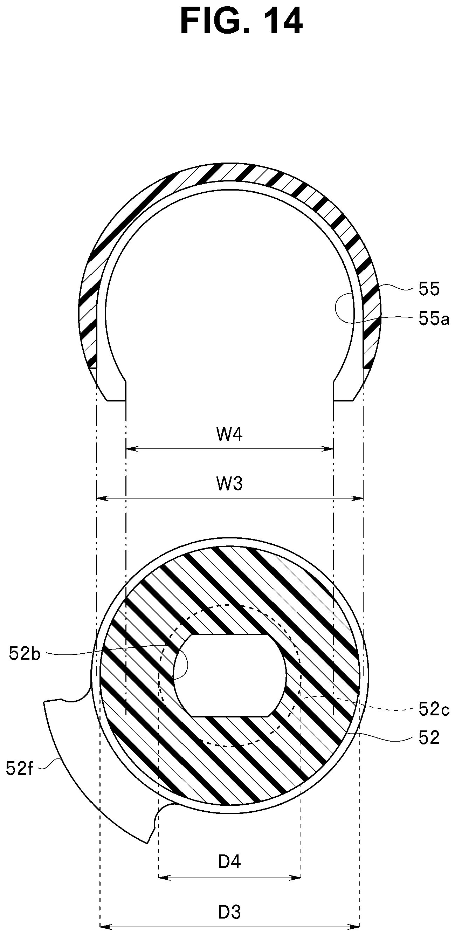

Here, for example, as shown in Fig. 14, a distance W3 between both end portions of the protective member 55 is set to be equal to or more than a diameter D3 of the lateral bending pulley 52. A distance W4 between both end portions of each projections 55a is set to be sufficiently greater than a diameter D4 of each of the pulley grooves 52c. Therefore, the protective member 55 can be attached to the lateral bending pulley 52 only by inserting the corner projections 55a having the arc shape into an outer circumference side of the pulley grooves 52c, respectively (see Fig. 13). That is, the protective member 55 can be inserted into the outer circumference side of the lateral bending pulley 52 without deforming the substantially C-shape of the protective member 55. Similarly, the projections 55a can be inserted into the outer circumference side of the pulley grooves 52c, respectively, without deforming the substantially C-shape of the projections 55a. Note that as another example, the protective member 55 and the respective projections 55a can be integrally formed by a material having elasticity. The protective member 55 and the projections 55a thus can be elastically deformed in a direction in which the both end portions in the circumference direction away from each other. With this, for example, even in a case where the distance W3 between the both end portions of the protective member 55 is set to be less than the diameter D3 of the lateral bending pulley 52, the distance W3 can be temporarily changed to equal to or more than the diameter D3 by the elastic deformation of the protective member 55. Therefore, the protective member 55 and the projections 55a can be mounted to the lateral bending pulley 52 and the pulley grooves 52c respectively from the outer circumference side of the lateral bending pulley 52 and the pulley grooves 52c.

As shown in Fig. 8, the projections 55a include shapes that fit into the pulley grooves 52c respectively when the protective member 55 is mounted to the lateral bending pulley 52. Therefore, the projections 55a are located on the same axis as the central axis O3 of the lateral bending pulley 52.

As shown in Figs. 8 to 10, the both end portions of the protective member 55 and each of the projections 55a in the circumference direction are located in a direction in which each of the lateral bending operation wires 38 extends from each of the pulley grooves 52c of the lateral bending pulley 52. Therefore, advance/retreat movement of each of the lateral bending operation wires 38 with rotation of the lateral bending pulley 52 is allowed.

In addition, the projections 55a that fit into the pulley grooves 52c respectively restrict the movement of the lateral bending operation wire 38 in the radially outward direction of the lateral bending pulley 52. Therefore, the protective member 55 prevents the lateral bending operation wire 38 from falling off the pulley groove 52c when being relaxed.

As above, in the present embodiment, the protective member 55 corresponds to a specific example of a cover. In addition, the projection 55al corresponds to a specific example of a third projection, and the projection 55ar corresponds to a specific example of a fourth projection.

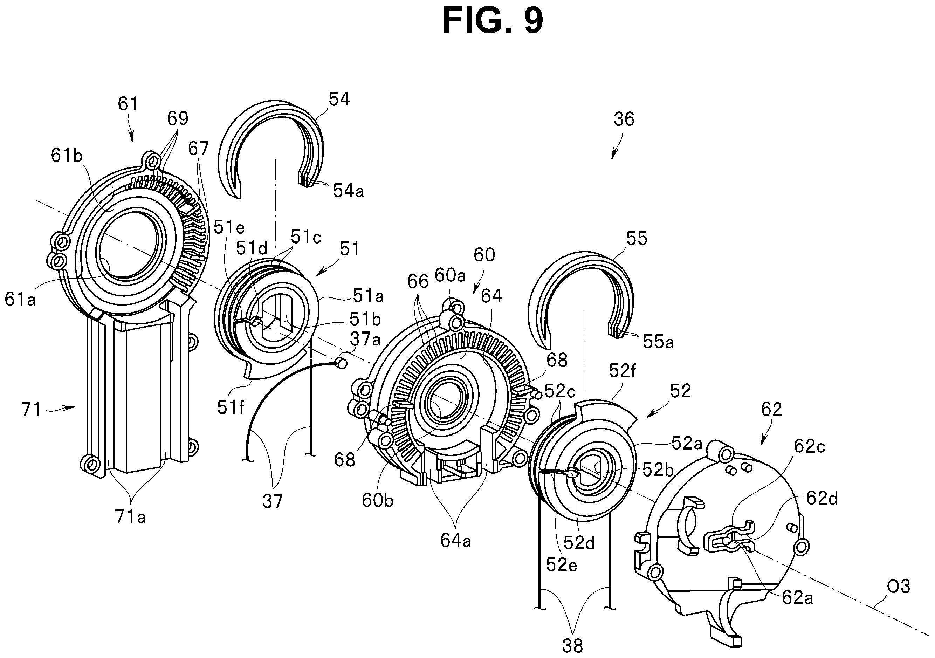

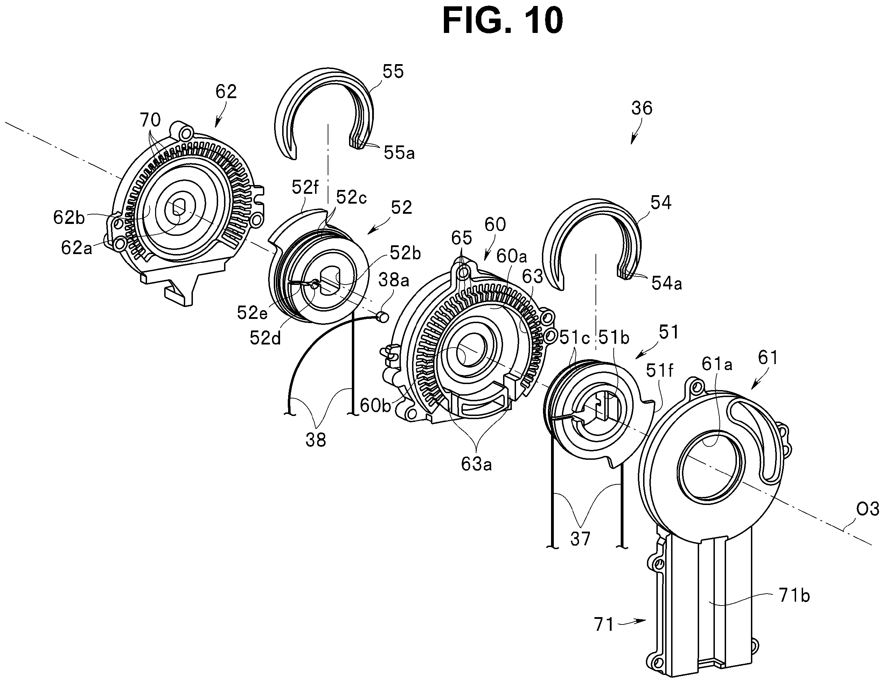

As shown in Figs. 9 and 10, the pulley case 53 includes a case main body 60, and a first case member 61 and a second case member 62 attached to both sides of the case main body 60.

The case main body 60 is formed of a member having a substantially cylindrical shape. An inner diameter of the case main body 60 is set to be larger than an outer diameter of each of the protective members 54 and 55, and smaller than an outer diameter of each of the convex portions 51f and 52f.

The case main body 60 includes a partition wall 60a inside thereof. The partition wall 60a divides the inside of the case main body 60 into a vertical bending pulley chamber 63 and a lateral bending pulley chamber 64. Furthermore, the partition wall 60a is provided with a shaft hole 60b that penetrates the partition wall 60a at a central portion thereof in the central axis O3 direction.

A depth of the vertical bending pulley chamber 63 (a length in the central axis O3 direction) is less than the thickness of the vertical bending pulley 51 by a predetermined dimension. Therefore, the vertical bending pulley chamber 63 can accommodate the vertical bending pulley 51 in a state where the convex portion 51f faces another end surface of the case main body 60.

The case main body 60 is provided with a pair of communication grooves 63a for allowing an inside to communicate with an outside of the vertical bending pulley chamber 63. The communication grooves 63a allow the vertical bending operation wire 37 wound in each of the pulley grooves 51c of the vertical bending pulley 51 to extend to the outside of the vertical bending pulley chamber 63.

The case main body 60 is provided with a plurality of mounting portions 65 on the other end surface in the central axis O3 direction. Each of the mounting portions 65 is, for example, configured by a slit-shaped recessed groove. Each of the mounting portions 65 is arranged radially around the central axis O3 of the case main body 60 with a predetermined distance between each other. To each of the mounting portions 65, a flat plate-shaped stopper member 67 made of metal or a resin can be detachably mounted.

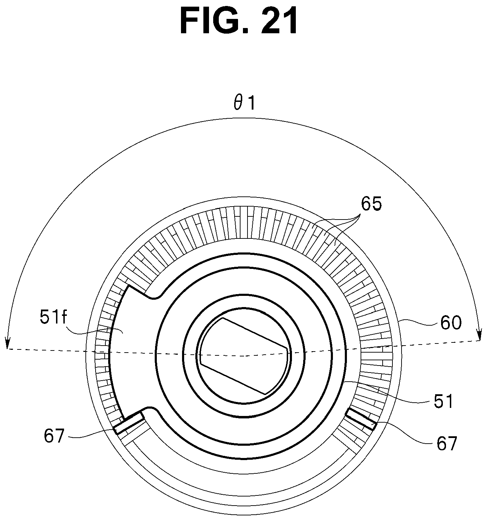

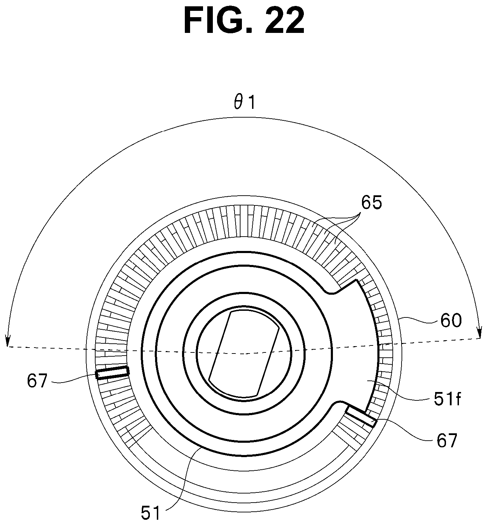

A group of the mounting portions 65 arranged in this way is selectively mounted with at least one stopper member 67. In the present embodiment, the group of the mounting portions 65 is selectively mounted with two stopper members 67. A part of the stopper members 67 mounted to the mounting portions 65 protrudes from the other end surface of the case main body 60. The protruded part of the stopper member 67 can contact the convex portion 51f of the vertical bending pulley 51. Therefore, each of the stopper members 67 restricts a rotation angle of the vertical bending pulley 51. That is, each of the stopper members 67 defines a rotatable angle θ1 of the vertical bending pulley 51 according to positions of the mounting portions 65 to which the stopper members 67 are selectively mounted respectively (for example, see Figs. 21 and 22).

In the present embodiment, the range of the rotatable angle θ1of the vertical bending pulley 51 is set to be from -180 degrees to +180 degrees with respect to an initial position of the vertical bending pulley 51. Here, the initial position of the vertical bending pulley 51 in a rotational direction is, for example, a rotation position when the bending portion 11 is not bent in the vertical direction. That is, the initial position of the vertical bending pulley 51 is, for example, a neutral position of the vertical bending pulley 51 when an operating force to the vertical bending operation knob 22 is released. In this case, when an absolute value of the rotation angle of the vertical bending pulley 51 is at its maximum on the negative side, the winding of one of the pair of the vertical bending operation wires 37 in the pulley groove 51c is less than one round. The winding of the other of the vertical bending operation wire 37 in the pulley groove 51c is one round or more. Conversely, when the absolute value of the rotation angle of the vertical bending pulley 51 is at its maximum on the positive side, the winding of one of the vertical bending operation wires 37 of the pair of the vertical bending operation wires 37 in the pulley groove 51c is one round or more. The winding of the other of the vertical bending operation wire 37 in the pulley groove 51c is less than one round.

A depth of the lateral bending pulley chamber 64 (a length in the central axis O3 direction) is less than the thickness of the lateral bending pulley 52 by a predetermined dimension. Therefore, the lateral bending pulley chamber 64 can accommodate the lateral bending pulley 52 in a state where the convex portion 52f faces one end surface of the case main body 60.

The case main body 60 is provided with a pair of communication grooves 64a for allowing an inside to communicate with an outside of the lateral bending pulley chamber 64. The communication grooves 64a allow the lateral bending operation wire 38 wound in each of the pulley grooves 52c of the lateral bending pulley 52 to extend to the outside of the lateral bending pulley chamber 64.

The case main body 60 is provided with a plurality of mounting portions 66 on the one end surface in the central axis O3 direction. Each of the mounting portions 66 is, for example, configured by a slit-shaped recessed groove. Each of the mounting portions 66 is arranged radially around the central axis O3 of the case main body 60 with a predetermined distance between each other. To each of the mounting portions 66, a flat plate-shaped stopper member 68 made of metal or a resin can be detachably mounted.

A group of the mounting portions 66 arranged in this way is selectively mounted with at least one stopper member 68. In the present embodiment, the group of the mounting portions 66 is selectively mounted with two stopper members 68. A part of the stopper members 68 mounted to the mounting portions 66 protrudes from the one end surface of the case main body 60. The protruded part of the stopper member 68 can contact the convex portion 52f of the lateral bending pulley 52. Therefore, each of the stopper members 68 restricts a rotation angle of the lateral bending pulley 52. That is, each of the stopper members 68 defines a rotatable angle θ2 of the lateral bending pulley 52 according to positions of the mounting portions 66 to which the stopper members 68 are selectively mounted respectively.

In the present embodiment, the range of the rotatable angle θ2 of the lateral bending pulley 52 is set to be from -180 degrees to +180 degrees with respect to an initial position of the lateral bending pulley 52. Here, the initial position of the lateral bending pulley 52 in a rotational direction is, for example, a rotation position when the bending portion 11 is not bent in the lateral direction. That is, the initial position of the lateral bending pulley 52 is, for example, a neutral position of the lateral bending pulley 52 when an operating force to the lateral bending operation knob 23 is released. In this case, when an absolute value of the rotation angle of the lateral bending pulley 52 is at its maximum on the negative side, the winding of one of the pair of the lateral bending operation wires 38 in the pulley groove 52c is less than one round. The winding of the other of the lateral bending operation wire 38 in the pulley groove 52c is one round or more. Conversely, when the absolute value of the rotation angle of the lateral bending pulley 52 is at its maximum on the positive side, the winding of one of the lateral bending operation wire 38 of the pair of the lateral bending operation wires 38 in the pulley groove 52c is one round or more. The winding of the other of the lateral bending operation wire 38 in the pulley groove 52c is less than one round.

The first case member 61 has a substantially disk shape. The first case member 61 has an outer diameter substantially the same as an outer diameter of the case main body 60. The first case member 61 is provided with a shaft hole 61a penetrating the first case member 61 at a central portion thereof in the central axis O3 direction.

The first case member 61 may include a recessed portion 61b that faces the vertical bending pulley 51 and is formed on one end surface (a surface on a side facing the other end surface of the case main body 60).

Furthermore, the first case member 61 is provided, on the one end surface, with mounting portions 69 corresponding respectively to the mounting portions 65 provided on the other end surface of the case main body 60.

The first case member 61 configured in this way is fixed to another end of the case main body 60 with screwing or the like. With this fixation, the vertical bending pulley 51 is held between the first case member 61 and the case main body 60. With this, the vertical bending pulley 51 is rotatably held inside the vertical bending pulley chamber 63.

At this time, the stopper member 67 is held in a state sandwiched between the mounting portions 65 and the mounting portions 69. Note that for simplifying the structure, it is possible to omit either the mounting portions 65 on the other end surface of the case main body 60 or the mounting portions 69 of the first case member 61.

In addition, the first case member 61 includes a bracket 71 integrally formed. The bracket 71 has a substantially rectangular plate shape that extends in a radially outward direction of the first case member 61.

Furthermore, the bracket 71 is provided with guide grooves 71a having a groove shape. The guide grooves 71a guide the pair of the vertical bending operation wires 37 extended from the vertical bending pulley chamber 63 and the pair of the lateral bending operation wires 38 extended from the lateral bending pulley chamber 64. Note that the protective member 54 mounted to the vertical bending pulley 51 and the protective member 55 mounted to the lateral bending pulley 52 are arranged on opposite sides of the guide grooves 71a in the circumference direction of the vertical bending pulley chamber 63 and the lateral bending pulley chamber 64.

The second case member 62 has a substantially disk shape. The second case member 62 has an outer diameter substantially the same as the outer diameter of the case main body 60. The second case member 62 is provided with a shaft hole 62a penetrating the second case member 62 at a central portion thereof in the central axis O3 direction.

The second case member 62 may include a recessed portion 62b that faces the lateral bending pulley 52 and is formed on another end surface (a surface on a side facing the one end surface of the case main body 60).

Furthermore, the second case member 62 is provided, on the other end surface, with mounting portions 70 corresponding respectively to mounting portions 66 provided on the one end surface of the case main body 60.

The second case member 62 configured in this way is fixed to one end of the case main body 60 with screwing or the like. With this fixation, the lateral bending pulley 52 is held between the second case member 62 and the case main body 60. With this, the lateral bending pulley 52 is rotatably held inside the lateral bending pulley chamber 64.

At this time, the stopper member 68 is held in a state sandwiched between the mounting portions 66 and the mounting portions 70. Note that for simplifying the structure, it is possible to omit either the mounting portions 66 on the other end surface of the case main body 60 or the mounting portions 70 of the second case member 62.

The pulley unit 36 configured in this way is fixed on an inner surface side of the first housing member 16 with screwing or the like. Specifically, the pulley unit 36 is, for example, fixed to the first housing member 16 using some of a plurality of screws for fixing the first case member 61 and the second case member 62 to the case main body 60. The pulley unit 36 is, for example, fixed to the first housing member 16 by screwing, the bracket 71 to the first housing member 16, or other method.

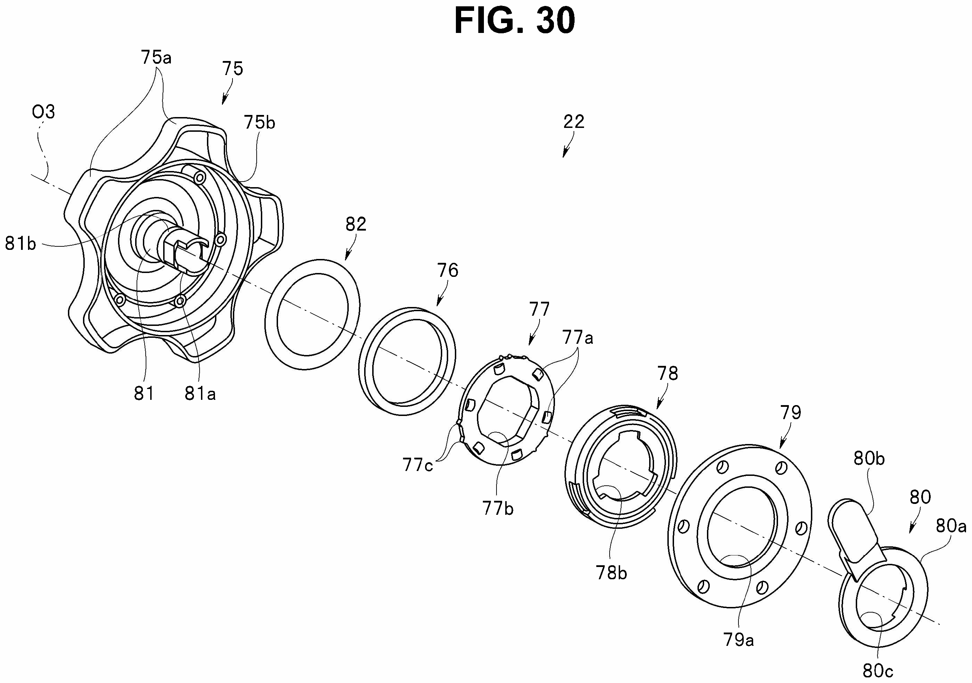

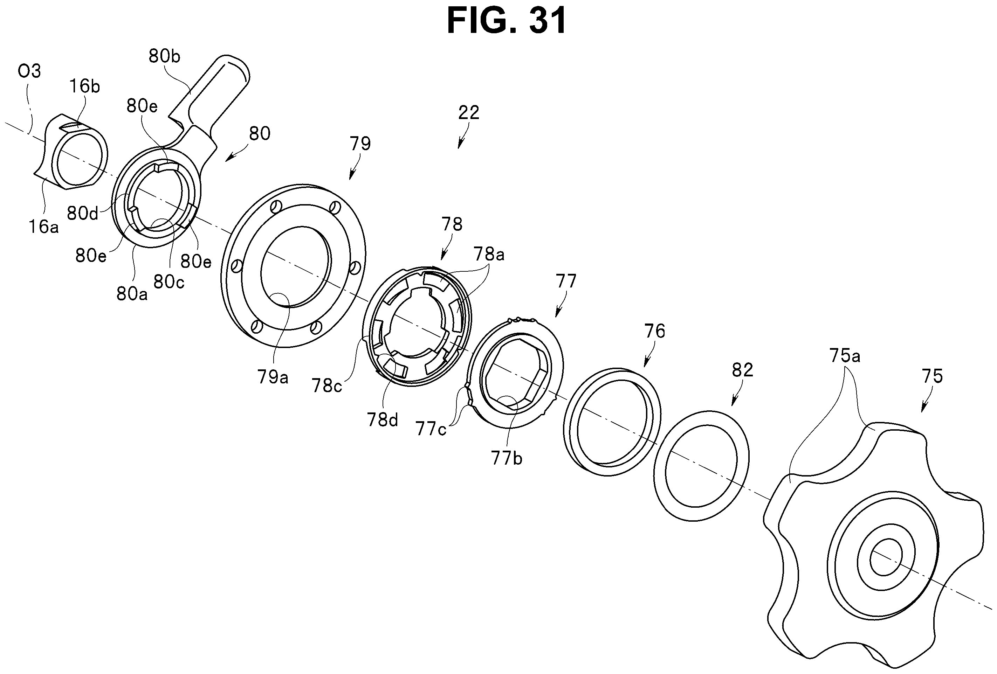

As shown in Figs. 8, 30, and 31, the vertical bending operation knob 22 is, for example, a break integrated bending operation knob. That is, the vertical bending operation knob 22 in the present embodiment integrally includes a break mechanism that holds a rotation position of the vertical bending operation knob 22.

The vertical bending operation knob 22 includes an operation knob main body 75, a friction rubber 76, a push plate 77, a cam plate 78, a lid body 79, a break operation lever 80, and a friction sheet 82.

The operation knob main body 75 is formed by a resin molding article, for example. The operation knob main body 75 includes a plurality of finger hooking portions 75a that radially protrude. The operation knob main body 75 includes a break chamber 75b formed on an inner side of the finger hooking portions 75a. The break chamber 75b is open on one end side in the central axis O3 direction and has a substantially cylindrical shape.

The operation knob main body 75 includes a hollow shaft 81 that protrudes on the one end side along the central axis O3 direction and is integrally formed at a central portion of the operation knob main body 75 (a central portion of the break chamber 75b).

The hollow shaft 81 is provided with a key 81a that can be fitted into the key hole 51b of the vertical bending pulley 51 at one end portion of the hollow shaft 81. The key 81a has a shape in which a part of an outer circumference portion of the hollow shaft 81 on the one end side is cut out, for example.

The hollow shaft 81 includes a fragile portion 81b formed at a middle portion. The fragile portion 81b is formed by providing a groove on an outer circumference of the hollow shaft 81, for example. With the fragile portion 81b, a torsional strength of the hollow shaft 81 is set to be weaker than a pulling strength of each of the vertical bending operation wires 37.

The friction rubber 76 is formed in an annular shape, for example. The friction rubber 76 is accommodated in the break chamber 75b via the annular friction sheet 82.

The push plate 77 is formed by a resin molding article, for example. The push plate 77 has a substantially disk shape. An outer diameter of the push plate 77 is set to be substantially the same as an outer diameter of the friction rubber 76.

The push plate 77 includes a plurality of cam followers 77a that are arc-shaped projections and are formed on one end surface of the push plate 77. The cam followers 77a are annularly arranged around the central axis O3 on the one end surface of the push plate 77.

The push plate 77 is provided with a key hole 77b that penetrates the push plate 77 at a central portion thereof in the central axis O3 direction.

Furthermore, the push plate 77 is provided with a plurality of minute outward projections 77c that protrude in a radially outward direction on an outer circumference portion of the push plate 77.

The push plate 77 configured in this way is accommodated in the break chamber 75b in a state where another end surface abuts on the friction rubber 76.

The cam plate 78 is formed by a resin molding article, for example. The cam plate 78 has a substantially disk shape. An outer diameter of the cam plate 78 is set to be larger than the outer diameter of the push plate 77.

The cam plate 78 includes a plurality of cams 78a formed on the other end surface in the central axis O3 direction. Each of the cams 78a is configured by a slope-shaped projection in which a protruded amount to another end side gradually changes along a circumferential direction. The cams 78a are annularly arranged to face the cam followers 77a respectively on the other end surface of the cam plate 78.

The cam plate 78 is provided with a key hole 78b that penetrates the cam plate 78 at a central portion thereof in the central axis O3 direction.

The cam plate 78 includes an annular flange 78c that protrudes to another end side and is formed on an outer edge portion. The flange 78c has an inner diameter larger than the outer diameter of the push plate 77.

The flange 78c is provided, on an inner circumference portion, with a plurality of minute inward projections 78d that protrude in a radially inward direction. The inward projections 78d can engage respectively with the outward projections 77c provided on the push plate 77.

The cam plate 78 configured in this way is accommodated in the break chamber 75b in a state where the push plate 77 is accommodated in an inner side of the flange 78c.

The lid body 79 is formed by a resin molding article, for example. The lid body 79 has a substantially disk shape. An outer diameter of the lid body 79 is set to be substantially the same as an inner diameter of the break chamber 75b.

The lid body 79 is provided with a through hole 79a that penetrates the lid body 79 in the central axis O3 direction.

The lid body 79 is fixed to the operation knob main body 75 by screwing or the like in a state accommodated in the break chamber 75b.

The break operation lever 80 is formed by a resin molding article, for example. The break operation lever 80 includes, for example, a rotation plate 80a having a substantially disk shape, and a lever 80b that protrudes in a radially outward direction of the rotation plate 80a.

The rotation plate 80a is provided with a through hole 80c that penetrates the rotation plate 80a at a central portion in the central axis O3 direction.

The rotation plate 80a includes an annular flange 80d that protrudes to another end side and is formed on an inner edge portion. An outer diameter of the flange 80d is set to be substantially the same as an inner diameter of the through hole 79a of the lid body 79. The flange 80d is slidably fitted into the through hole 79a of the lid body 79.

Furthermore, a key 80e that can fit into the key hole 78b of the cam plate 78 protrudes from a part of the flange 80d. The key 80e is key-fitted into the key hole 78b of the cam plate 78, so that the break operation lever 80 can integrally rotate with the cam plate 78.

Here, in the vertical bending operation knob 22, the hollow shaft 81 protrudes to one end side with respect to the break operation lever 80 through the central portions of the friction sheet 82, the friction rubber 76, the push plate 77, the cam plate 78, the lid body 79, and the break operation lever 80.

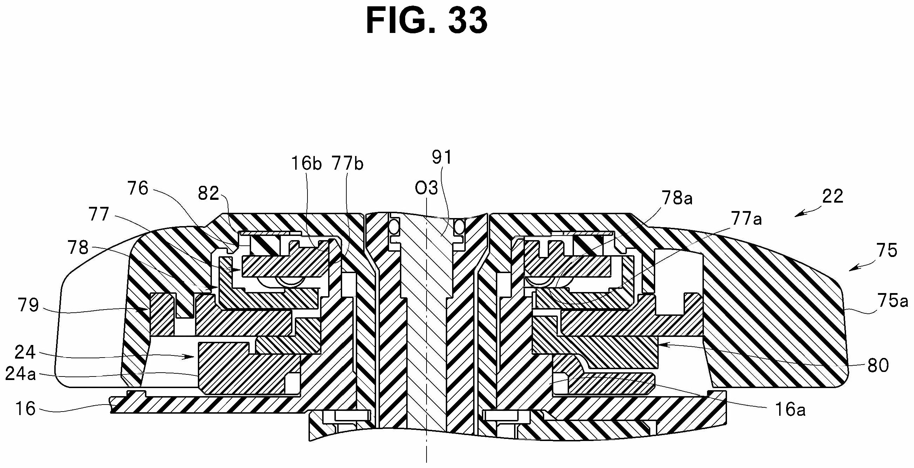

As shown in Figs. 3, 8, 32, and 33, the vertical bending operation knob 22 configured in this way is attached to the first housing member 16 through a shaft cylinder 16a that protrudes from a side portion of the first housing member 16.

Specifically, the hollow shaft 81 of the vertical bending operation knob 22 is rotatably inserted through an inside of the shaft cylinder 16a. With this, the vertical bending operation knob 22 is rotatably supported by the first housing member 16.

In this case, a key 16b provided in one end portion of the shaft cylinder 16a is fitted into the key hole 51b of the vertical bending pulley 51. With this, the vertical bending pulley 51 can rotate in conjunction with a rotating operation to the vertical bending operation knob 22. Then, according to the rotation state, the vertical bending pulley 51 pulls or relaxes the pair of the vertical bending operation wires 37. Therefore, the vertical bending operation knob 22 can cause the bending portion 11 to bend in the vertical direction via the pulley unit 36.

Note that at the time of such bending operation, when an excessive operating force is applied to the vertical bending pulley 51, the fragile portion 81b of the hollow shaft 81 is destroyed before each of the vertical bending operation wires 37 are broken. With this, each of the vertical bending operation wires 37 is prevented from breaking.

Here, a protruded end portion of the shaft cylinder 16a (another end portion of the shaft cylinder 16a in the central axis O3 direction) that protrudes from the first housing member 16 is provided with the key 16b. The key 16b has, for example, a shape in which a part of an outer circumference portion of the shaft cylinder 16a on the other end side is cut out. The key 16b passes through the break operation lever 80, the lid body 79, and the cam plate 78 and is key-fitted into the key hole 77b of the push plate 77. With this, the push plate 77 is non-rotatably supported by the shaft cylinder 16a (first housing member 16).

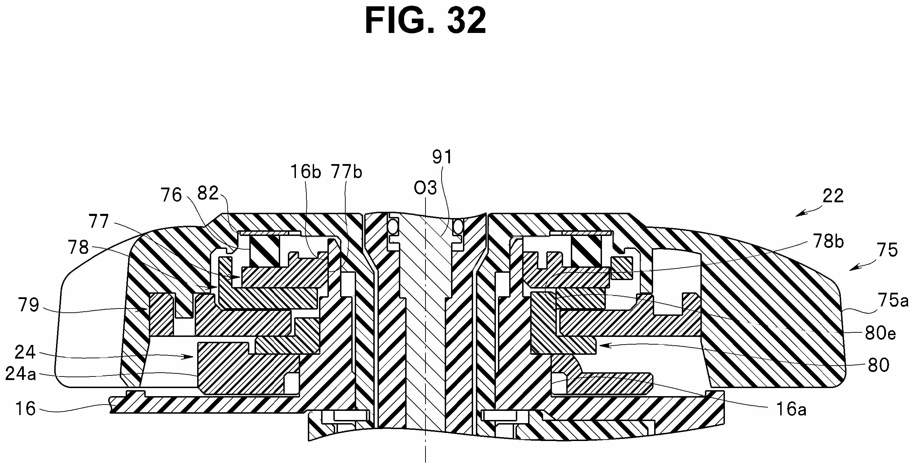

When the cam plate 78 is rotated with an operation to the break operation lever 80, the push plate 77 rotates relatively to the cam plate 78. With the relative rotation, an abutting position of the cam follower 77a with respect to the cam 78a changes. With the change in the abutting position of the cam follower 77a and the cam 78a, the push plate 77 is displaced to the operation knob main body 75 side (see a change from Fig. 32 to Fig. 33). With this, the friction rubber 76 is pressed against the friction sheet 82 and the operation knob main body 75 while being elastically deformed under a pressing force of the push plate 77. Then, the pressing force of the push plate 77 generates a strong friction force between the operation knob main body 75 and the friction sheet 82 and between the cam plate 78 and the lid body 79. Here, the push plate 77 is not rotatable with respect to the shaft cylinder 16a. These restrict rotation of the operation knob main body 75. This restriction of the rotation of the operation knob main body 75 holds a rotation position of the vertical bending operation knob 22 (the break is operated). Note that once the rotation position of the vertical bending operation knob 22 is held and thereafter, if the vertical bending operation knob 22 is forcibly rotated, the cam plate 78 also rotates, the pressing force of the push plate 77 decreases, and there is a possibility that the friction force decreases. The inward projections 78d of the flange 78c are engaged respectively with the outward projections 77c provided on the push plate 77, to prevent the cam plate 78 from rotating. Therefore, even if the vertical bending operation knob 22 whose rotation position is held is forcibly rotated, the rotation of the cam plate 78 is restricted. As a result, the pressing force of the push plate 77 does not decrease, and the friction force does not decrease either.

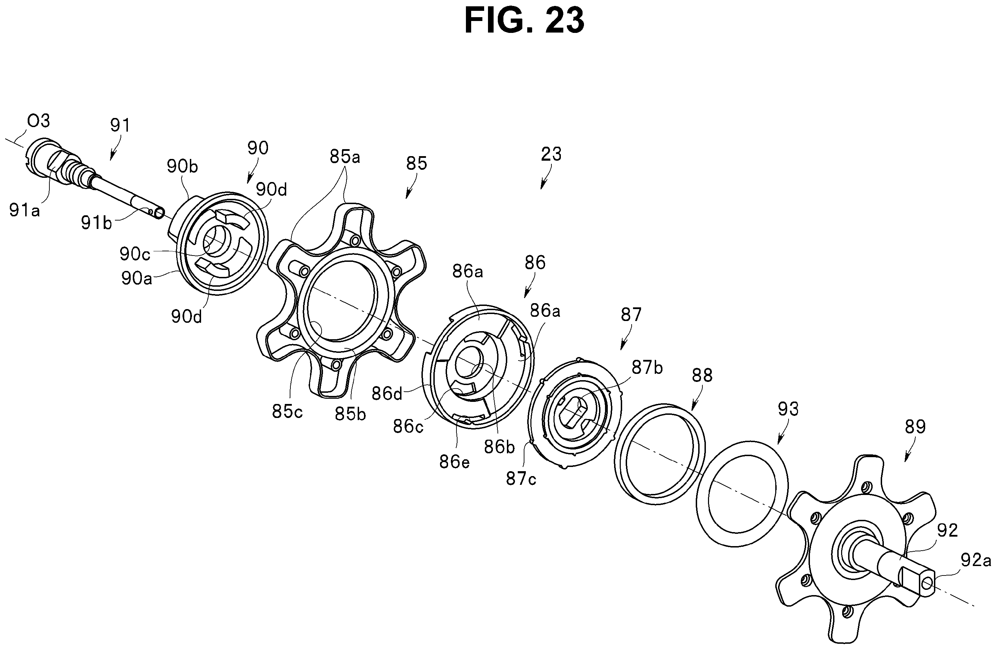

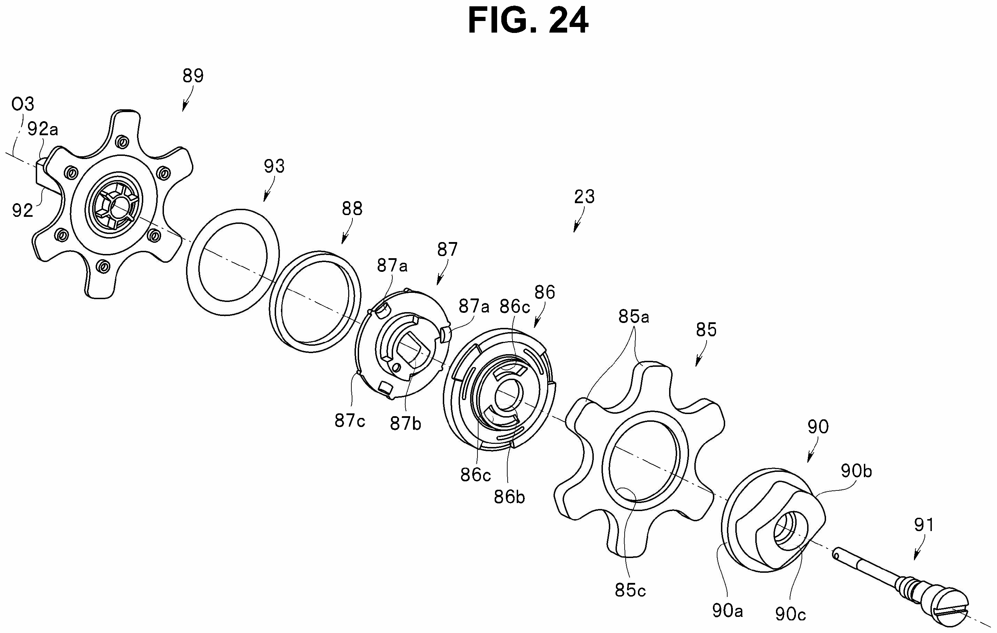

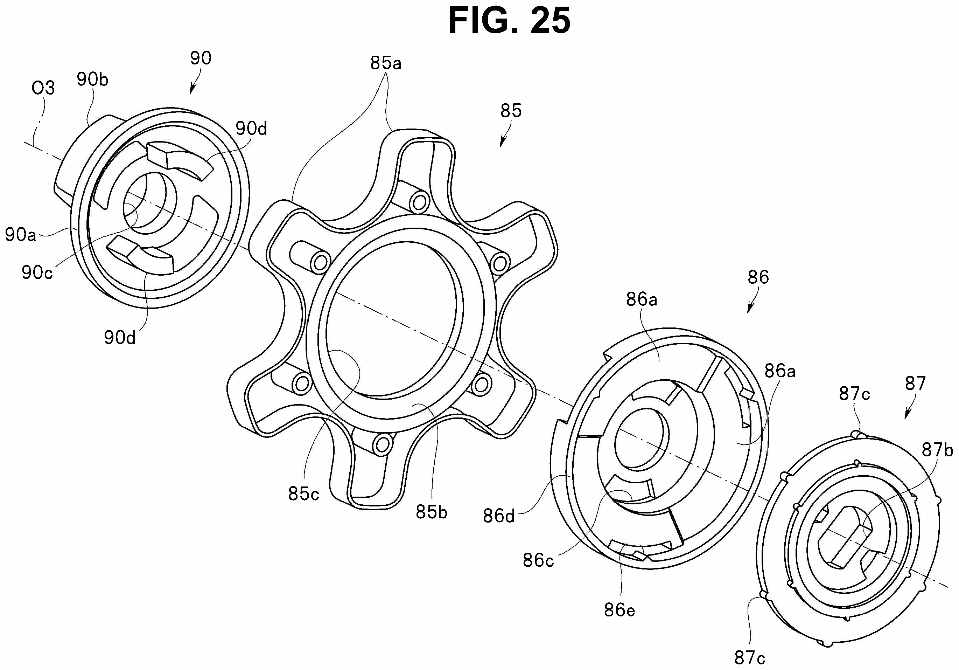

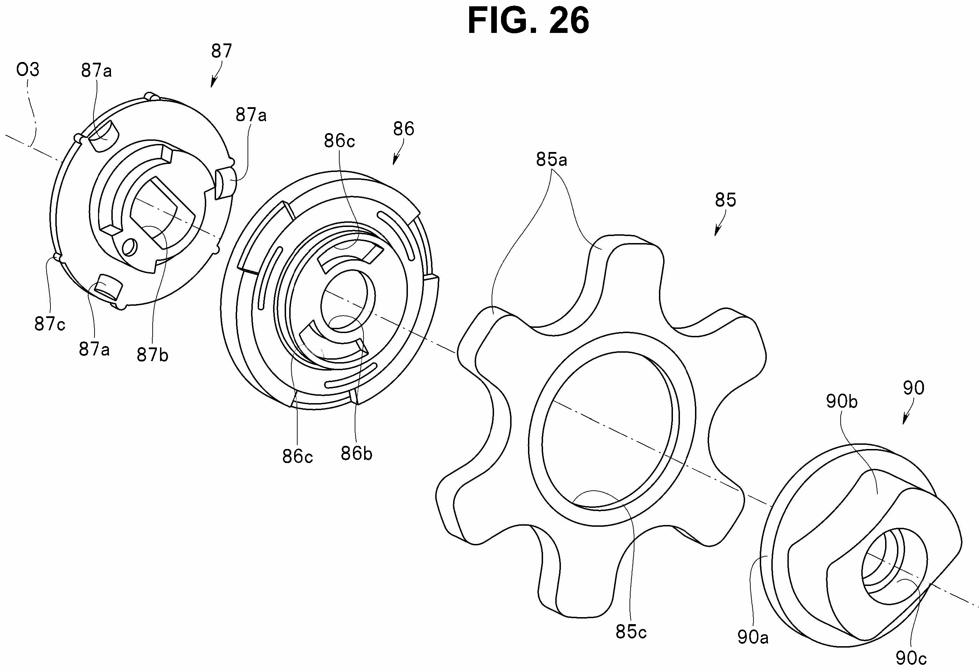

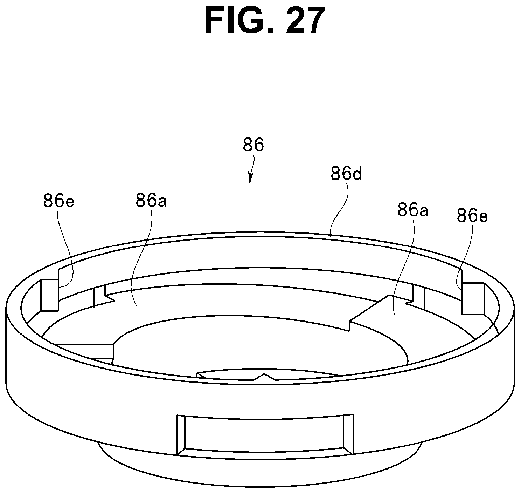

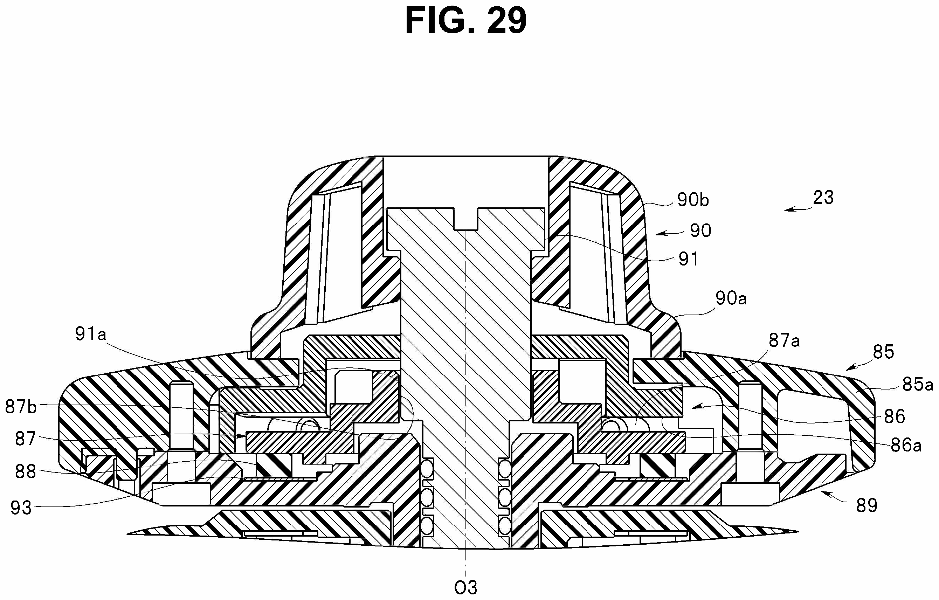

As shown in Figs. 8, and 23 to 29, the lateral bending operation knob 23 is, for example, a break integrated bending operation knob. That is, the lateral bending operation knob 23 in the present embodiment integrally includes a break mechanism that holds a rotation position of the lateral bending operation knob 23.

The lateral bending operation knob 23 includes an operation knob main body 85, a cam plate 86, a push plate 87, a friction rubber 88, a lid body 89, a break operation knob 90, a fixing shaft 91, and a friction sheet 93.

The operation knob main body 85 is formed by a resin molding article, for example. The operation knob main body 85 includes a plurality of finger hooking portions 85a that radially protrude. The operation knob main body 85 includes a break chamber 85b formed on an inner side of the finger hooking portions 85a. The break chamber 85b is open on one end side in the central axis O3 direction.

The operation knob main body 85 is provided with a through hole 85c that penetrates the operation knob main body 85 at a central portion thereof in the central axis O3 direction.

The cam plate 86 is formed by a resin molding article, for example. The cam plate 86 has a substantially disk shape.

The cam plate 86 includes a plurality of cams 86a formed on one end surface in the central axis O3 direction. Each of the cams 86a is configured by a slope-shaped projection in which a protruded amount to one end side gradually changes along a circumferential direction. The cams 86a are annularly arranged around the central axis O3 on the one end surface of the cam plate 86.

The cam plate 86 is provided with a through hole 86b that penetrates the cam plate 86 at a central portion thereof in the central axis O3 direction. Furthermore, the cam plate 86 is provided, around the through hole 86b, with a key hole 86c that penetrates the cam plate 86 in the central axis O3 direction.

The cam plate 86 includes an annular flange 86d that protrudes to one end side and is formed on an outer edge portion. The flange 86d is provided, on an inner circumference portion, with a plurality of minute inward projections 86e that protrude in a radially inward direction.

The cam plate 86 configured in this way is accommodated in the break chamber 85b.

The push plate 87 is formed by a resin molding article, for example. The push plate 87 has a substantially disk shape. An outer diameter of the push plate 87 is set to be smaller than an inner diameter of the flange 86d of the cam plate 86.

The push plate 87 includes a plurality of cam followers 87a that are arc-shaped projections and are formed on another end surface of the push plate 87. The cam followers 87a are annularly arranged to face cams 86a respectively on the other end surface of the push plate 87.

The push plate 87 is provided with a key hole 87b that penetrates the push plate 87 at a central portion in the central axis O3 direction.

Furthermore, the push plate 87 is provided with a plurality of minute outward projections 87c that protrude in a radially outward direction on an outer circumference portion of the push plate 87. The outward projections 87c can be engaged respectively with the inward projections 86e provided on the cam plate 86.

The push plate 87 configured in this way is accommodated in an inner side of the flange 86d of the cam plate 86.

The friction rubber 88 is formed in an annular shape, for example. The friction rubber 88 is accommodated in the break chamber 85b in a state abutted on the push plate 87.

The lid body 89 is formed by a resin molding article, for example. The lid body 89 has a substantially flat plate shape similar to a plan view shape of the operation knob main body 85.

The lid body 89 includes an integrally formed hollow shaft 92 that protrudes to one end side along the central axis O3 direction.

The hollow shaft 92 is provided with a key 92a that can be fitted into the key hole 52b of the lateral bending pulley 52 at one end portion of the hollow shaft 92. The key 92a has a shape in which a part of an outer circumference portion of the hollow shaft 92 on the one end side is cut out, for example

The key 92a is set as a fragile portion that elastically deforms when an external force of a predetermined level or more is applied. A torsional strength of the key 92a is set to be weaker than a pulling strength of each of the lateral bending operation wires 38.

The lid body 89 is fixed to the operation knob main body 85 by screwing or the like in a state abutted on one end side of the operation knob main body 85. In this case, the friction rubber 88 abuts on the lid body 89 via the annular friction sheet 93.

The break operation knob 90 is formed by a resin molding article, for example. The break operation knob 90 includes a rotation member 90a having a substantially gentle truncated cone shape, and a knob 90b that protrudes to another end side of the rotation member 90a.

The break operation knob 90 is provided with a through hole 90c that penetrates the rotation member 90a and the knob 90b at a central portion of the break operation knob 90 in the central axis O3 direction.

The rotation member 90a includes a key 90d that can be fitted into the key hole 86c of the cam plate 86 and protrudes from one end surface of the rotation member 90a.

The rotation member 90a of the break operation knob 90 rotatably abuts on the other end side of the operation knob main body 85. In this state, the key 90d of the break operation knob 90 is key-fitted into the key hole 86c of the cam plate 86 through the through hole 85c of the operation knob main body 85. With this, the break operation knob 90 can rotate integrally with the cam plate 86.

The fixing shaft 91 is formed by a metal cutting product, for example. At a middle portion of the fixing shaft 91, a key 91a that can be fitted into the key hole 87b of the push plate 87 is provided.

The fixing shaft 91 is inserted through an inside of the hollow shaft 92 through the central portions of the break operation knob 90, the operation knob main body 85, the cam plate 86, the push plate 87, the friction rubber 88, and the friction sheet 93. In this case, the key 91a of the fixing shaft 91 is key-fitted into the key hole 87b of the push plate 87.

As shown in Fig. 8, the lateral bending operation knob 23 configured in this way is attached to the first housing member 16 by inserting the hollow shaft 92 of the lateral bending operation knob 23 through the hollow shaft 81 of the vertical bending operation knob 22.

In this case, the key 92a provided in one end portion of the hollow shaft 92 is fitted into the key hole 52b of the lateral bending pulley 52. With this, the lateral bending pulley 52 can rotate in conjunction with the rotating operation to the lateral bending operation knob 23. According to the rotation state, the lateral bending pulley 52 pulls or relaxes the pair of the lateral bending operation wires 38. Therefore, the lateral bending operation knob 23 can cause the bending portion 11 to bend in the lateral direction via the pulley unit 36.

Note that at the time of such bending operation, when an excessive operating force is applied to the lateral bending operation knob 23, the key 92a of the hollow shaft 92 is destroyed before each of the lateral bending operation wires 38 are broken. With this, each of the lateral bending operation wires 38 is prevented from breaking.

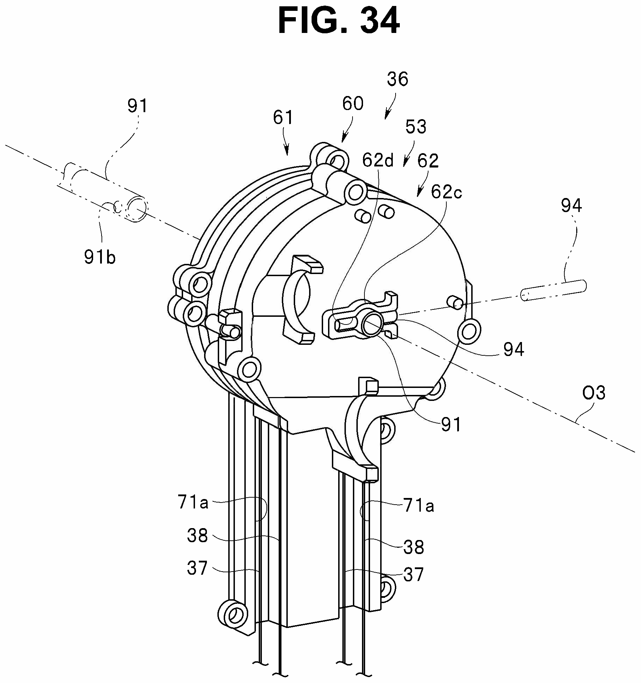

Here, as shown in Figs. 8 and 34, one end portion of the fixing shaft 91 inserted through the hollow shaft 92 is non-rotatably coupled to the shaft hole 62a of the second case member 62.

Specifically, the fixing shaft 91 includes a pin hole 91b in one end portion.
The pin hole 91b is a hole that penetrates the fixing shaft 91 in a direction orthogonal to the central axis O3. The pin hole 91b is provided at a position that is exposed to the outside of the pulley case 53 from one end surface of the second case member 62 when the fixing shaft 91 is inserted through the hollow shaft 92. A fixing pin 94 is inserted through the pin hole 91b exposed from the second case member 62. This prevents the fixing shaft 91 from coming off the pulley case 53.

Furthermore, as shown in Figs. 9 and 34, the second case member 62 includes a pin receiver 62c on one end surface (outer surface) of the second case member 62. The pin receiver 62c is, for example, configured by a projection including a groove 62d that can accommodate the fixing pin 94. The groove 62d of the pin receiver 62c extends in a direction orthogonal to the central axis O3. In addition, the groove 62d of the pin receiver 62c extends in a direction orthogonal to a direction in which a pulling force of each of the vertical bending operation wires 37 and each of the lateral bending operation wires 38 are applied. The pin receiver 62c restricts rotation of the fixing pin 94 inserted through the pin hole 91b around the central axis O3. With this, the push plate 87 is non-rotatably supported with respect to the shaft cylinder 16a (first housing member 16). In this case, the fixing pin 94 held by the groove 64d extends in a direction orthogonal to a direction in which the pulling force of each of the vertical bending operation wires 37 and each of the lateral bending operation wires 38 are applied. Therefore, the fixing pin 94 suppress the occurrence of shaking of the fixing shaft 91 or the like, due to the pulling force of each of the vertical bending operation wires 37 and each of the lateral bending operation wires 38.

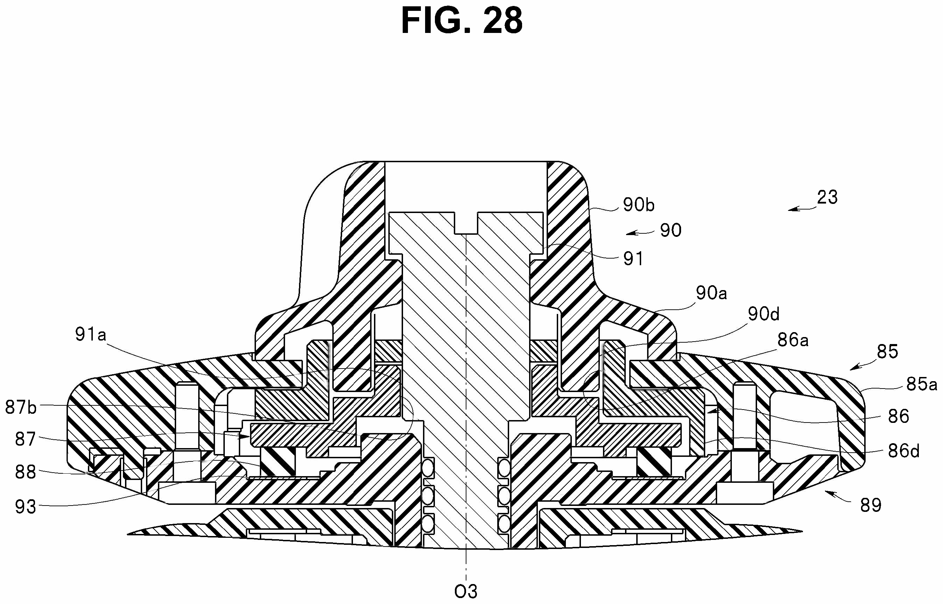

When the cam plate 86 is rotated with an operation to the break operation knob 90, the push plate 87 rotates relatively to the cam plate 86. With the relative rotation, an abutting position of the cam follower 87a with respect to the cam 86a changes. With the change in the abutting position of the cam follower 87a with respect to the cam 86a, the push plate 87 is displaced to the lid body 89 side (see a change from Fig. 28 to Fig. 29). With this, the friction rubber 88 is pressed against the friction sheet 93 and the lid body 89 while elastically deforming under a pressing force of the push plate 87. Then, the pressing force of the push plate 87 generates a strong friction force between the lid body 89 and the friction sheet 93 and between the cam plate 86 and the operation knob main body 85. Here, the push plate 87 is not rotatable with respect to the fixing shaft 91. Furthermore, the lid body 89 is non-rotatably fixed with respect to the operation knob main body 85. These restrict rotation of the operation knob main body 85. This restriction of the rotation with respect to the operation knob main body 85 holds a rotation position of the lateral bending operation knob 23. Note that once the rotation position of the lateral bending operation knob 23 is held and thereafter, if the lateral bending operation knob 23 is forcibly rotated, the cam plate 86 also rotates, the pressing force of the push plate 87 decreases, and there is a possibility that the friction force decreases. The inward projections 86e of the flange 86d are engaged respectively with the outward projections 87c provided on the push plate 87, to prevent the cam plate 86 from rotating. Therefore, even if the lateral bending operation knob 23 whose rotation position is held is forcibly rotated, the rotation of the cam plate 86 is restricted. As a result, the pressing force of the push plate 87 does not decrease, and the friction force does not decrease either.

Next, a configuration of the raising base operation mechanism 40 will be described in detail.

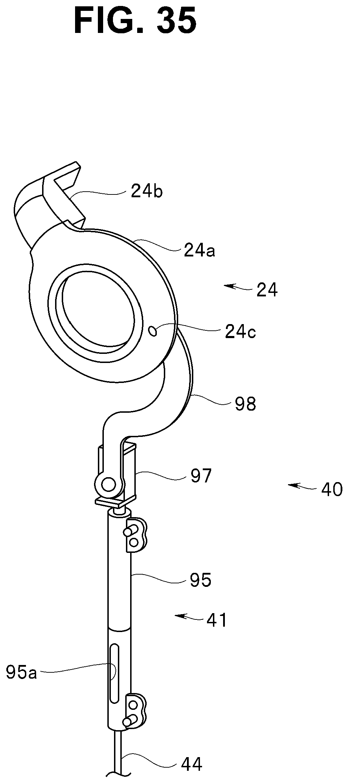

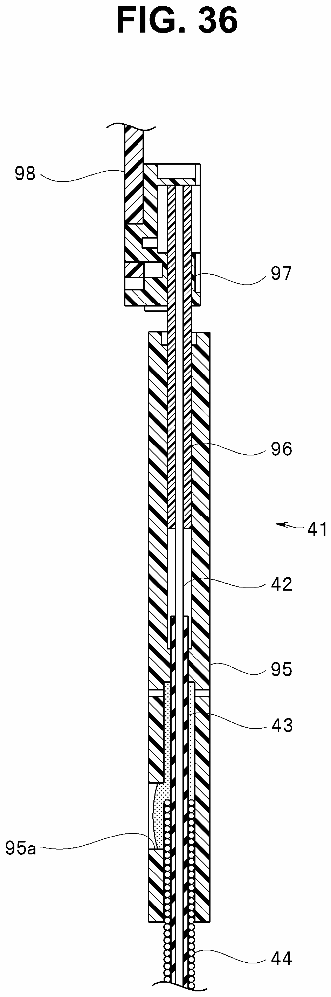

As shown in Figs. 35 and 36, the cylinder unit 41 of the raising base operation mechanism 40 includes a cylinder 95 through which a proximal end side of the raising base operation wire 42 can be inserted. A rod 96 is inserted into the proximal end side of the cylinder 95 so as to advance and retreat.

The rod 96 is connected to a proximal end portion of the raising base operation wire 42. Specifically, the proximal end side of the raising base operation wire 42 is inserted through an inside of the cylinder 95 from a distal end side of the cylinder 95. Inside the cylinder 95, the proximal end portion of the raising base operation wire 42 is exposed from a sheath 43. Then the proximal end portion of the raising base operation wire 42 exposed from the sheath 43 is connected to the rod 96. Outside the cylinder 95, a head member 97 is connected to a proximal end side of the rod 96. Here, the head member 97 is configured to be slidable with respect to the recessed portion 71b (see Fig. 10) provided in the bracket 71. With this, the head member 97 is guided to the recessed portion 71b, to be movable along the longitudinal axis O2 direction of the housing 15.

In a side portion of the cylinder 95 on the distal end side, a communication hole 95a that allows communication between an inside and an outside of the cylinder 95 is provided. In the vicinity of the communication hole 95a, a proximal end side of a guide coil 44 that covers the sheath 43 of the raising base operation wire 42 is inserted inside the cylinder 95. By injecting an adhesive from the communication hole 95a to the inside of the cylinder 95, the guide coil 44 and the sheath 43 are adhered and fixed to an inner circumferential surface of the cylinder 95.

The raising base operation lever 24 includes an annular plate-shaped rotation cam 24a, and a lever 24b that protrudes in a radially outward direction of the rotation cam 24a. The rotation cam 24a and the lever 24b are integrally formed by resin molding, for example.

As shown in Fig. 8, the rotation cam 24a is arranged between the vertical bending operation knob 22 and the first housing member 16. The shaft cylinder 16a is inserted through the rotation cam 24a. With this, the raising base operation lever 24 is rotatably supported by the first housing member 16.

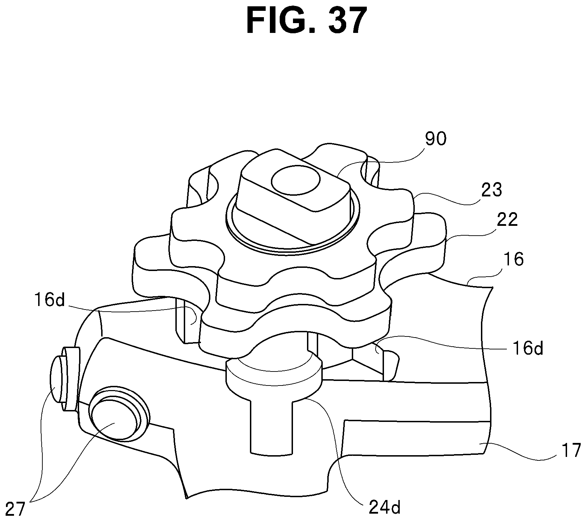

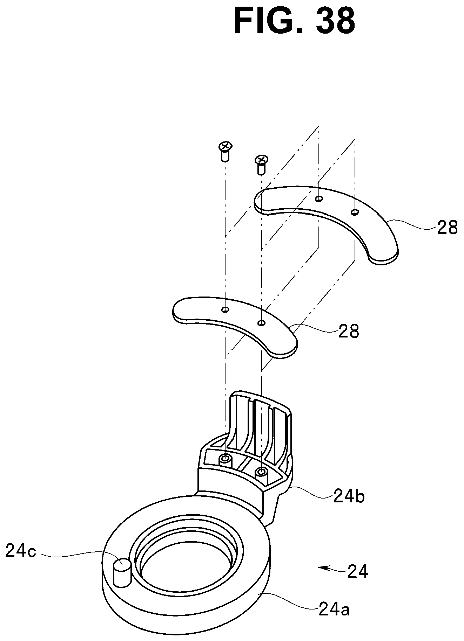

Here, the rotation cam 24a is provided with a cam pin 24c that protrudes on the first housing member 16 side. The cam pin 24c is inserted through an arc-shaped key hole 16c provided in the first housing member 16. Rotation of the raising base operation lever 24 is, for example, restricted in an angle range α within which the lever 24b abuts on two projections 16d formed on the first housing member 16 (see Fig. 37). Here, the angle range α in which the rotation of the raising base operation lever 24 is allowed can be adjusted by attaching an angle adjustment plate 28 to the lever 24b, for example. As shown in Fig. 38, the angle adjustment plate 28 is prepared in a plurality of patterns in advance according to a model or the like of the endoscope 1. The angle adjustment plate 28 is attached to the lever 24b with screwing or the like, as appropriate.

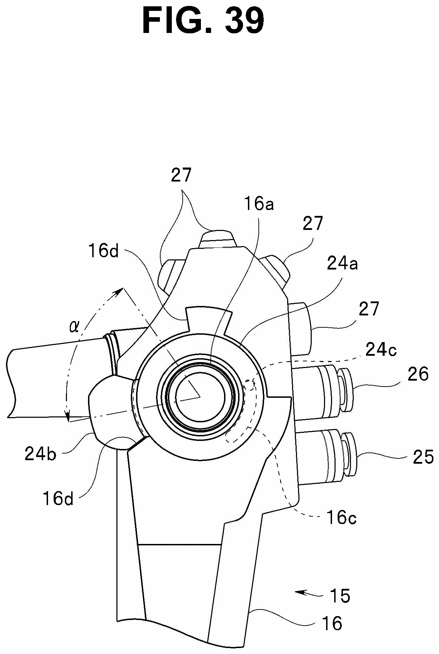

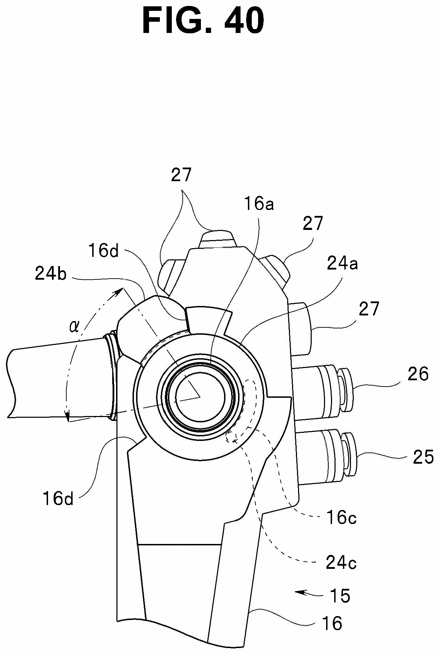

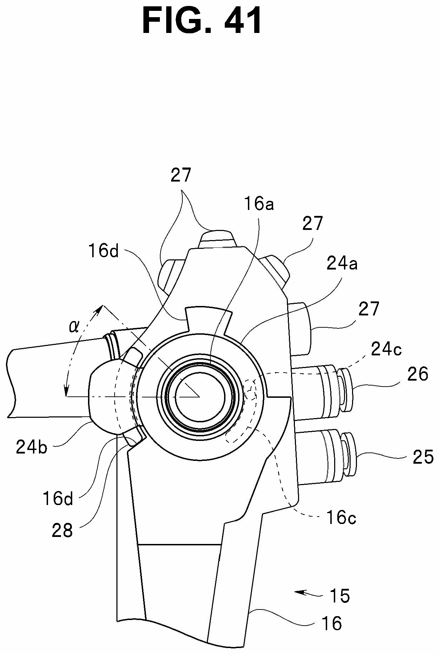

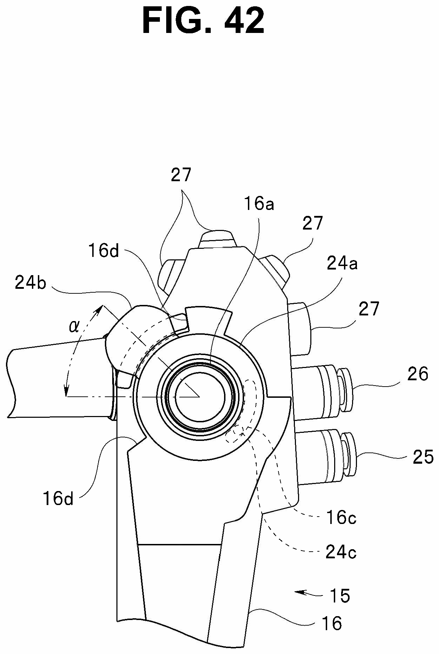

As above, for example, as shown in Figs. 39 to 42, the angle range α when the angle adjustment plate 28 is attached is restricted to be smaller than the angle range α when the angle adjustment plate 28 is not attached. That is, when the angle adjustment plate 28 is attached, an end portion of the angle adjustment plate 28 abuts on the projection 16d before the lever 24b abuts on the projection 16d. Therefore, the angle range α when the angle adjustment plate 28 is attached is restricted.

Inside the first housing member 16, the cam pin 24c is connected to the head member 97 of the cylinder unit 41 via a relay member 98. With this, the rod 96 advances and retreats in the cylinder 95 in conjunction with the rotating operation to the raising base operation lever 24. The advance/retreat movement of the rod 96 causes the raising base operation wire 42 to be pulled or relaxed. With this, the raising base 10c provided in the distal end portion 10 can displace between a raising position and a laid position.

According to the above embodiment, the endoscope 1 includes the pair of the vertical bending operation wires 37 for transmitting the pulling force to the bending portion 11 of the insertion portion 5, the vertical bending pulley 51 in which the pair of the pulley grooves 51c for winding each of the vertical bending operation wires 37 are provided in the outer circumference portion, and the wire guides 51g that are provided in the partial region in the circumference direction of each of the pulley grooves 51c, and that guide, to the predetermined winding position, each of the vertical bending operation wires 37 to be wound in the each of the pulley grooves 51c. The angle at which the vertical bending pulley 51 can rotate is 180 degrees or less, and the bottom portion of each of the pulley grooves 51c has the width that allows each of the vertical bending operation wires 37 to be wound around for at most two rounds. The width of the bottom portion of the pulley groove 51c in the partial region is narrowed down with each of the wire guides 51g so that each of the vertical bending operation wires 37 can be wound only one round in the rotation axis direction of the vertical bending pulley 51, and each of the wire guides 51g has a tapered shape to guide each of the vertical bending operation wires 37 to be wound in the partial region to the bottom portion of each of the pulley grooves 51c.

Similarly, the endoscope 1 includes the pair of the lateral bending operation wires 38 for transmitting the pulling force to the bending portion 11 of the insertion portion 5, the lateral bending pulley 52 in which the pair of the pulley grooves 52c for winding each of the lateral bending operation wires 38 are provided in the outer circumference portion, and the wire guides 52g that are provided in the partial region in the circumference direction of each of the pulley grooves 52c, and that guide, to the predetermined winding position, each of the lateral bending operation wires 38 to be wound in the each of the pulley grooves 52c. The angle at which the lateral bending pulley 52 can rotate is 180 degrees or less, and the bottom portion of each of the pulley grooves 52c has the width that allows each of the lateral bending operation wires 38 to be wound around for at most two rounds. The width of the bottom portion of the pulley groove 52c in the partial region is narrowed down with each of the wire guides 52g so that each of the lateral bending operation wires 38 can be wound only one round in the rotation axis direction of the lateral bending pulley 52, and each of the wire guides 52g has a tapered shape to guide each of the lateral bending operation wires 38 to be wound in the partial region to the bottom portion of each of the pulley grooves 52c.

With these, the vertical bending operation wires 37 and the lateral bending operation wires 38 can be wound in the pulley grooves 51c of the vertical bending pulley 51 and the pulley grooves 52c of the lateral bending pulley 52, respectively, in an appropriate state.

That is, the width of the bottom portion in the partial region in the circumference direction of the pulley groove is narrowed down with the wire guide. Then, when the bending operation wire that is displaced in a direction away from the pulley groove due to relaxing is wound in the pulley groove, the wire guide guides the bending operation wire to the bottom portion of the pulley groove with the narrowed width. Therefore, when the winding state of the bending operation wire in the pulley groove is changed from the first round to the second round, the bending operation wire can be properly prevented from being wound in the pulley groove in a state where the bending operation wire overlaps in the radial direction of the pulley.

In this case, the wire guide is provided at a position adjacent to the starting position for winding of the bending operation wire in the pulley groove. Furthermore, the wire guide extends in a direction opposite to the winding direction of the bending operation wire in the pulley groove. With these, the wire guide can properly guide the bending operation wire when the winding state in the pulley groove is changed from the first round to the second round.

In addition, the endoscope 1 includes the protective member to cover the pulley groove from the outer circumference side of the bending pulley. Furthermore, the protective member includes the projection protruded toward the inside of the pulley groove from at least part in the inner circumference surface of the protective member. With these configurations, when the bending operation wire is relaxed, the bending operation wire is properly prevented from being separated from the pulley groove. Therefore, when the relaxed bending operation wire is wound in the pulley groove, the bending operation wire can be properly prevented from being wound in the other adjacent pulley groove.

Note that the present invention is not limited to the above described embodiment, and various modifications and alternations can be made and those are in the technical scope of the present invention.

For example, the endoscope bending operation mechanism shown in the above described embodiment has the configuration for bending the bending portion in the vertical direction and the lateral direction, but is obviously not limited to such a configuration. For example, the endoscope bending operation mechanism may be configured to bend the bending portion in either the vertical or lateral direction only.

The present application claims priority to U.S. Provisional Application No. 63/623,861, filed in the United States on January 23, 2024, and the disclosure therein is incorporated by reference to the specification, claims, and drawings of the present application.

### List of Reference Signs

1: endoscope, 5: insertion portion, 6: operation portion, 7: universal cord, 8: endoscope connector, 8a: liquid feeding connector, 8b: gas feeding connector, 8c: engaging claw, 9: relay connector, 9a: control substrate, 9b: light source connector, 9c: gas feeding plug, 9d: electric contact point, 9e: connector receiving hole, 10: distal end portion, 10a: flat portion, 10b: opening portion, 10c: raising base, 10d: illumination window, 10e: observation window, 10f: nozzle, 11: bending portion, 11a: bending piece, 11b: coupling member, 11c: outer sheath, 11d: thickened portion, 12: flexible tube portion, 15: housing, 16: first housing member, 16a: shaft cylinder, 16b: key, 16c: key hole, 16d: projection, 17: second housing member, 20: grasping portion, 21: channel port, 22: vertical bending operation knob, 23: lateral bending operation knob, 24: raising base operation lever, 24a: rotation cam, 24b: lever, 24c: cam pin, 25: gas/liquid feeding button, 26: suction button, 27: button switch, 28: angle adjustment plate, 30: branch tube, 31: treatment instrument channel, 32: first suction tube, 35: bending operation mechanism, 36: pulley unit, 37: vertical bending operation wire, 37d: vertical bending operation wire, 37u: vertical bending operation wire, 38: lateral bending operation wire, 38d: lateral bending operation wire, 38u: lateral bending operation wire, 40: raising base operation mechanism, 41: cylinder unit, 42: raising base operation wire, 43: sheath, 44: guide coil, 45: gas/liquid feeding cylinder, 46a: first gas feeding tube, 46b: first liquid feeding tube, 46c: second gas feeding tube, 46d: second liquid feeding tube, 47: gas/liquid feeding tube, 48: suction cylinder, 49: second suction tube, 51: vertical bending pulley, 51a: vertical bending pulley main body, 51b: key hole, 51c: pulley groove, 51cd: pulley groove, 51cu: pulley groove, 51d: wire stopper receiving hole, 51e: connection groove, 51f: convex portion, 51g: wire guide, 51gd: wire guide, 51gu: wire guide, 52: lateral bending pulley, 52a: lateral bending pulley main body, 52b: key hole, 52c: pulley groove, 52cu: pulley groove, 52cd: pulley groove, 52cl: pulley groove, 52cr: pulley groove, 52d: receiving hole, 52e: connection groove, 52f: convex portion, 52g: wire guide, 52gd: wire guide, 52gu: wire guide, 53: pulley case, 54: protective member, 54a: projection, 54ad: projection, 54au: projection, 55: protective member, 55a: projection, 55al: projection, 55ar: projection, 60: case main body, 60a: partition wall, 60b: shaft hole, 61: first case member, 61a: shaft hole, 61b: recessed portion, 62: second case member, 62a: shaft hole, 62b: recessed portion, 62d: groove, 63: vertical bending pulley chamber, 63a: communication groove, 64: lateral bending pulley chamber, 64a: communication groove, 64d: groove, 65: mounting portion, 66: mounting portion, 67: stopper member, 68: stopper member, 69: mounting portion, 70: mounting portion, 71: bracket, 71a: guide groove, 71b: recessed portion, 75: operation knob main body, 75a: finger hooking portion, 75b: brake chamber, 76: friction rubber, 77: push plate, 77a: cam follower, 77b: key hole, 77c: outward projection, 78: cam plate, 78a: cam, 78b: key hole, 78c: flange, 78d: inward projection, 79: lid body, 79a: through hole, 80: brake operation lever, 80a: rotation plate, 80b: lever, 80c: through hole, 80d: flange, 80e: key, 81: hollow shaft, 81a: key, 81b: fragile portion, 82: friction sheet, 85: operation knob main body, 85a: finger hooking portion, 85b: brake chamber, 85c: through hole, 86: cam plate, 86a: cam, 86b: through hole, 86c: key hole, 86d: flange, 86e: inward projection, 87: push plate, 87a: cam follower, 87b: key hole, 87c: outward projection, 88: friction rubber, 89: lid body, 90: brake operation knob, 90a: rotation member, 90b: knob, 90c: through hole, 90d: key, 91: fixing shaft, 91a: key, 91b: pin hole, 92: hollow shaft, 92a: key, 93: friction sheet, 94: fixing pin, 95: cylinder, 95a: communication hole, 96: rod, 97: head member, 98: relay member

## Claims

1. An insertion device comprising:
a wire configured to transmit a pulling force to a bending portion of an insertion portion configured to be inserted into a subject;
a pulley including a groove for winding the wire on an outer circumference portion of the pulley; and
a wire guide that is provided in a partial region in a circumference direction of the groove, and is configured to guide the wire to be wound in the groove to a predetermined winding position, wherein:
an angle range within which the pulley can rotate is from -180 degrees to +180 degrees with respect to an initial position of the pulley; and
a width of a bottom portion of the groove in the partial region is narrowed down with the wire guide so that the wire can be wound only one round in a direction of a rotation axis of the pulley, and the wire guide has a tapered shape to guide the wire to be wound in the partial region to the bottom portion of the groove.

2. The insertion device according to claim 1, wherein the bottom portion of the groove has a width that allows the wire to be wound for at most two rounds in the direction of the rotation axis of the pulley.

3. The insertion device according to claim 1, wherein the wire guide is provided at a position adjacent to a starting position for winding of the wire in the groove.

4. The insertion device according to claim 3, wherein the wire guide extends in a direction opposite to a winding direction of the wire in the groove.

5. The insertion device according to claim 1, further comprising a cover that covers the groove from an outer circumference side of the pulley, wherein
the cover includes a projection that protrudes toward an inside of the groove from at least a part in an inner circumference surface of the cover.

6. The insertion device according to claim 5, wherein:
the inner circumference surface of the cover has an arc shape that is coaxial with the rotation axis of the pulley; and
a tip shape of the projection has an arc shape that is coaxial with the rotation axis of the pulley.

7. The insertion device according to claim 5, wherein the projection has a shape that fits into an inside of the groove.

8. The insertion device according to claim 6, wherein the projection has a C-shape when seen in a direction along the rotation axis of the pulley.

9. The insertion device according to claim 8, wherein a central angle of the projection having the arc shape is more than 180 degrees.

10. The insertion device according to claim 8, wherein both end portions in a circumference direction of the projection having the arc shape are arranged in a direction in which the wire extends from the groove of the pulley.

11. The insertion device according to claim 9, wherein the cover having the arc shape is formed of a material that can be elastically deformed in a direction in which both end portions in a circumference direction are separated from each other.

12. The insertion device according to claim 9, wherein the cover is attached to the pulley by inserting the projection having the arc shape into an outer circumference of the groove of the pulley.

13. The insertion device according to claim 1, wherein a diameter of the wire is 0.25 mm or more and 0.55 mm or less.

14. The insertion device according to claim 1, wherein the insertion device is a single-use endoscope disposed of after single use.

15. An insertion device comprising:
a first wire, a second wire, a third wire, and a fourth wire each configured to transmit a pulling force to a bending portion of an insertion portion configured to be inserted into a subject;
a first pulley and a second pulley, the first pulley including a first groove and a second groove for winding the first wire and the second wire respectively, on an outer circumference portion of the first pulley, the second pulley including a third groove and a fourth groove for winding the third wire and the fourth wire respectively, on an outer circumference portion of the second pulley; and
a first wire guide, a second wire guide, a third wire guide, and a fourth wire guide that are provided in a partial region in a circumference direction of each of the first groove, the second groove, the third groove, and the fourth groove, and are configured to respectively guide the first wire, the second wire, the third wire, and the fourth wire to be wound in the first groove, the second groove, the third groove, and the fourth groove, respectively, to predetermined winding positions, wherein:
angle ranges within which the first pulley and the second pulley can rotate are from -180 degrees to +180 degrees with respect to respective initial positions of the first pulley and the second pulley; and
widths of bottom portions of the first groove, the second groove, the third groove, and the fourth groove in the partial region are narrowed down with the first wire guide, the second wire guide, the third wire guide, and the fourth wire guide, respectively, so that the first wire, the second wire, the third wire, and the fourth wire can be wound only one round in a direction of a rotation axis of the first pulley or the second pulley, and the first wire guide, the second wire guide, the third wire guide, and the fourth wire guide have a tapered shape to guide the first wire, the second wire, the third wire, and the fourth wire to be wound in the partial region to the bottom portions of the first groove, the second groove, the third groove, and the fourth groove respectively.

16. The insertion device according to claim 15, wherein the bottom portions of the first groove, the second groove, the third groove, and the fourth groove have a width that allows the first wire, the second wire, the third wire, and the fourth wire to be wound for at most two rounds in the direction of the rotation axis of the first pulley or the second pulley.

17. The insertion device according to claim 15, further comprising:
a first projection that faces the first groove and restricts a movement range of the first wire with respect to a radially outward direction of the first pulley;
a second projection that faces the second groove and restricts a movement range of the second wire with respect to the radially outward direction of the first pulley;
a third projection that faces the third groove and restricts a movement range of the third wire with respect to a radially outward direction of the second pulley; and
a fourth projection that faces the fourth groove and restricts a movement range of the fourth wire with respect to the radially outward direction of the second pulley.

18. The insertion device according to claim 17, wherein:
the first projection is disposed adjacent to the second projection; and
the third projection is disposed adjacent to the fourth projection.

19. The insertion device according to claim 18, wherein:
the first projection and the second projection are formed integrally with a first cover that covers the first groove and the second groove from an outer circumference side of the first pulley; and
the third projection and the fourth projection are formed integrally with a second cover that covers the third groove and the fourth groove from an outer circumference side of the second pulley.
